# EUROPEAN PATENT APPLICATION

(11) **EP 4 241 713 A1**
(43) Date of publication of application: **13.09.2023**
(21) Application number: 22382234.7
(22) Date of filing: 11.03.2022
(51) Int. Cl.: A61B 18/14, A61B 90/00

(54) **ELECTROSURGICAL INSTRUMENT WITH CLAMP CLOSURE SENSOR**

(71) Applicant: Cilag GmbH International, 6300 Zug (CH)
(72) Inventor: De Córdoba, José Luis, 6300 Zug (CH)
(74) Representative: Carpmaels & Ransford LLP

(57) **Abstract**

An electrosurgical instrument includes a shaft assembly, an end effector, an input actuator, a biasing feature, an end of stroke feature, a first clamp closure sensor, a second clamp closure sensor, and a control unit. The end effector includes an energized feature, a first jaw, and a second jaw. The first clamp closure sensor is configured to produce first sensor information relating to movement of the input actuator. The second clamp closure sensor is configured to produce second sensor information relating to deflection of the biasing feature in response to a tissue load force between the first and second jaws. Based on each of the first and second sensor information, the control unit is configured to determine the tissue load force exerted on the end effector, and a position of one of the first and second jaws relative to the other of the first and second jaws.

## Description

### BACKGROUND

A variety of surgical instruments include a tissue cutting element and one or more elements that transmit radio frequency (RF) energy to tissue (e.g., to coagulate or seal the tissue). Examples of devices and related concepts are disclosed in U.S. Pat. No. 6,500,176 entitled "Electrosurgical Systems and Techniques for Sealing Tissue," issued December 31, 2002, the disclosure of which is incorporated by reference herein, in its entirety; U.S. Pat. No. 8,939,974, entitled "Surgical Instrument Comprising First and Second Drive Systems Actuatable by a Common Trigger Mechanism," issued January 27, 2015, the disclosure of which is incorporated by reference herein, in its entirety; U.S. Pat. No. 8,888,809, entitled "Surgical Instrument with Jaw Member," issued November 18, 2014, the disclosure of which is incorporated by reference herein, in its entirety; U.S. Pat. No. 9,161,803, entitled "Motor Driven Electrosurgical Device with Mechanical and Electrical Feedback," issued October 20, 2015, the disclosure of which is incorporated by reference herein, in its entirety; U.S. Pat. No. 9,877,720, entitled "Control Features for Articulating Surgical Device," issued January 30, 2018, the disclosure of which is incorporated by reference herein, in its entirety; U.S. Pat. No. 9,545,253, entitled "Surgical Instrument with Contained Dual Helix Actuator Assembly," issued January 17, 2017, the disclosure of which is incorporated by reference herein, in its entirety; and U.S. Pat. No. 9,526,565, entitled "Electrosurgical Devices," issued December 27, 2016, the disclosure of which is incorporated by reference herein, in its entirety.

Some electrosurgical instruments include an end effector with at least one compliant feature. Examples of such instruments are described in U.S. Pat. No. 9,149,325, entitled "End Effector with Compliant Clamping Jaw," issued October 6, 2015, the disclosure of which is incorporated by reference herein, in its entirety; and U.S. Pat. No. 9,877,782, entitled "Electrosurgical Instrument End Effector with Compliant Electrode," issued January 30, 2018, the disclosure of which is incorporated by reference herein, in its entirety.

While a variety of surgical instruments have been made and used, it is believed that no one prior to the inventors has made or used the invention described in the appended claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an exemplary electrosurgical instrument;
FIG. 2 depicts a perspective view of an exemplary articulation assembly and end effector of the electrosurgical instrument of FIG. 1;
FIG. 3 depicts an exploded view of the articulation assembly and end effector of FIG. 2;
FIG. 4A depicts a side elevational view of a handle assembly of the electrosurgical instrument of FIG. 1, where the end effector is in an open and unfired state, where a portion of the handle assembly is omitted for purposes of clarity;
FIG. 4B depicts a side elevational view of the handle assembly of FIG. 4A, where the end effector is in a closed and unfired state, where a portion of the handle assembly is omitted for purposes of clarity;
FIG. 4C depicts a side elevational view of the handle assembly of FIG. 4A, where the end effector is in a closed and fired state, where a portion of the handle assembly is omitted for purposes of clarity;
FIG. 5A depicts a cross-sectional side view of the end effector of FIG. 2, where the end effector is in the open and unfired state, taken along line 5-5 of FIG. 2;
FIG. 5B depicts a cross-sectional side view of the end effector of FIG. 2, where the end effector is in a partially closed and unfired state, taken along line 5-5 of FIG. 2 with tissue disposed between the jaws of the end effector;
FIG. 5C depicts a cross-sectional side view of the end effector of FIG. 2, where the end effector is in the closed and unfired state, taken along line 5-5 of FIG. 2;
FIG. 5D depicts a cross-sectional side view of the end effector of FIG. 2, where the end effector is in the closed and fired state, taken along line 5-5 of FIG. 2;
FIG. 6A depicts a side elevational view of a handle assembly of a first exemplary alternative electrosurgical instrument similar to the electrosurgical instrument of FIG. 1, where the end effector of FIG. 5A is in an open and unfired state, where the yoke assembly is shown in cross-section along a centerline thereof and a portion of the handle assembly is omitted for purposes of clarity;
FIG. 6B depicts a side elevational view of the handle assembly of FIG. 6A, but where the end effector of FIG. 5B is in a partially closed and unfired state;
FIG. 6C depicts a side elevational view of the handle assembly of FIG. 6B, but where the end effector of FIG. 5D is in a closed and fired state;
FIG. 7A depicts a side elevational view an enlarged portion of the handle assembly of FIG. 6A;
FIG. 7B depicts a side elevational view an enlarged portion of the handle assembly of FIG. 6B;
FIG. 7C depicts a side elevational view an enlarged portion of the handle assembly of FIG. 6C;
FIG. 8 depicts an enlarged perspective view of a portion of the handle assembly of FIG. 6A showing an exemplary clamp closure sensor;
FIG. 9 depicts an enlarged perspective view of a portion of the handle assembly of FIG. 8;
FIG. 9A depicts a sectional perspective view of the portion of the handle assembly of FIG. 9 taken along line 9A-9A of FIG. 9;
FIG. 10 depicts a perspective view of the clamp closure sensor of FIG. 8;
FIG. 11A depicts a side elevational view of a handle assembly of a second exemplary alternative electrosurgical instrument, where the end effector is in an open and unfired state, where a portion of the handle assembly is omitted for purposes of clarity;
FIG. 11B depicts a side elevational view of the handle assembly of FIG. 11A, where the end effector is in a partially closed and unfired state;
FIG. 11C depicts a side elevational view of the handle assembly of FIG. 11B, where the end effector is in a closed and fired state;
FIG. 12A depicts a cross-sectional side view of an end effector configured for use with the electrosurgical instrument of FIG. 11A, where the end effector is in the open and unfired state;
FIG. 12B depicts a cross-sectional side view of the end effector of FIG. 11A, but in a partially closed and unfired state with tissue disposed between the jaws of the end effector;
FIG. 12C depicts a cross-sectional side view of the end effector of FIG. 11A, but in the closed and fired state;
FIG. 13 depicts a side elevational view a portion a handle assembly of a third exemplary alternative electrosurgical instrument;
FIG. 14 depicts a graph that includes plots of handle position relative to jaw load for the electrosurgical instrument of FIG. 13;
FIG. 15 depicts a graph that includes plots of handle position relative to jaw load for various loading conditions for the electrosurgical instrument of FIG. 13;
FIG. 16 depicts a graph that includes plots of handle position, biasing feature position, and jaw position of unloaded jaws and loaded jaws without tissue compression;
FIG. 17 depicts a graph that includes plots of handle position, biasing feature position, and jaw position of unloaded jaws and loaded jaws with tissue compression;
FIG. 18 depicts a graph that includes plots of handle position, biasing feature position, and jaw position during a pre-energy application phase, an energy application phase, and a post-energy application phase;
FIG. 19 depicts a graph that includes plots of handle position, biasing feature position, and jaw position as tissue slips out from between overstuffed jaws; and
FIG. 20 depicts a diagrammatic view of an exemplary method of operating the electrosurgical instruments of FIGS. 6A, 11A, and 13.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a surgeon or other operator grasping a surgical instrument having a distal surgical end effector. The term "proximal" refers the position of an element closer to the surgeon or other operator and the term "distal" refers to the position of an element closer to the surgical end effector of the electrosurgical instrument and further away from the surgeon or other operator.

### I. Exemplary Electrosurgical Instrument

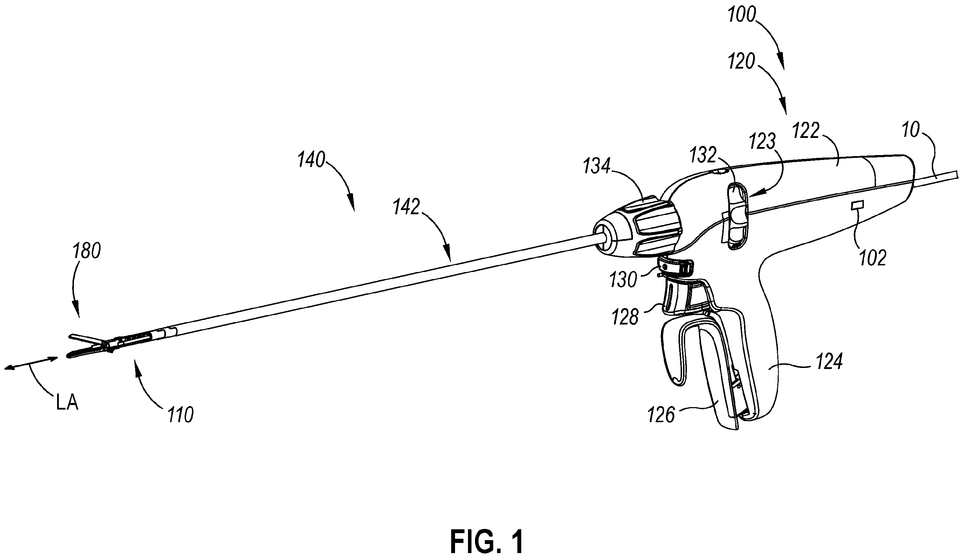

FIGS. 1-3C show an exemplary electrosurgical instrument (100). As best seen in FIG. 1, electrosurgical instrument (100) includes a handle assembly (120), a shaft assembly (140), an articulation assembly (110), and an end effector (180). As will be described in greater detail below, end effector (180) of electrosurgical instrument (100) is operable to grasp, cut, and seal or weld tissue (e.g., a blood vessel, etc.). In this example, end effector (180) is configured to seal or weld tissue by applying bipolar radio frequency (RF) energy to tissue. However, it should be understood electrosurgical instrument (100) may be configured to seal or weld tissue through any other suitable means that would be apparent to one skilled in the art in view of the teachings herein. For example, electrosurgical instrument (100) may be configured to seal or weld tissue via an ultrasonic blade, staples, etc. In the present example, electrosurgical instrument (100) is electrically coupled to a power source (not shown) via power cable (10).

The power source may be configured to provide all or some of the electrical power requirements for use of electrosurgical instrument (100). Any suitable power source may be used as would be apparent to one skilled in the art in view of the teachings herein. For example, the power source may be constructed in accordance with at least some of the teachings of U.S. Pat. No. 8,986,302, entitled "Surgical Generator for Ultrasonic and Electrosurgical Devices," issued March 24, 2015, the disclosure of which is incorporated by reference herein, in its entirety. While in the current example, electrosurgical instrument (100) is coupled to a power source via power cable (10), electrosurgical instrument (100) may contain an internal power source or plurality of power sources, such as a battery and/or supercapacitors, to electrically power electrosurgical instrument (100). Of course, any suitable combination of power sources may be utilized to power electrosurgical instrument (100) as would be apparent to one skilled in the art in view of the teaching herein.

Handle assembly (120) is configured to be grasped by an operator with one hand, such that an operator may control and manipulate electrosurgical instrument (100) with a single hand. Shaft assembly (140) extends distally from handle assembly (120) and connects to articulation assembly (110). Articulation assembly (110) is also connected to a proximal end of end effector (180). As will be described in greater detail below, components of handle assembly (120) are configured to control end effector (180) such that an operator may grasp, cut, and seal or weld tissue. Articulation assembly (110) is configured to deflect end effector (180) from the longitudinal axis (LA) defined by shaft assembly (140).

Handle assembly (120) includes a control unit (102) housed within a body (122), a pistol grip (124), a jaw closure trigger (126), a knife trigger (128), an activation button (130), an articulation control (132), and a knob (134). Jaw closure trigger (126) is operatively connected to end effector (180) and configured to selectively move from an unactuated position to an actuated position to move jaws (182, 184) from the open configuration toward the closed configuration. As will be described in greater detail below, jaw closure trigger (126) may be pivoted toward and away from pistol grip (124) and/or body (122) to open and close jaws (182, 184) of end effector (180) to grasp tissue. Additionally, knife trigger (128) may be pivoted toward and away from pistol grip (124) and/or body (122) to actuate a knife member (176) within the confines of jaws (182, 184) to cut tissue captured between jaws (182, 184). Further, activation button (130) may be pressed to apply radio frequency (RF) energy to tissue via an electrode assembly (193). As shown, electrode assembly (193) includes electrode surfaces (194, 196) of jaws (182, 184), respectively.

Body (122) of handle assembly (120) defines an opening (123) in which a portion of articulation control (132) protrudes from. Articulation control (132) is rotatably disposed within body (122) such that an operator may rotate the portion of articulation control (132) protruding from opening (123) to rotate the portion of articulation control (132) located within body (122). Rotation of articulation control (132) relative to body (122) is configured to bend articulation assembly (110) in order to drive deflection of end effector (180) from the longitudinal axis (LA) defined by shaft assembly (140). Articulation control (132) and articulation assembly (110) may include any suitable features to drive deflection of end effector (180) from the longitudinal axis (LA) defined by shaft assembly (140) as would be apparent to one skilled in the art in view of the teachings herein.

Knob (134) is rotatably disposed on the distal end of body (122) and configured to rotate end effector (180), articulation assembly (110), and shaft assembly (140) about the longitudinal axis (LA) of shaft assembly (140) relative to handle assembly (120). While in the current example, end effector (180), articulation assembly (110), and shaft assembly (140) are rotated by knob (134), knob (134) may be configured to rotate end effector (180) and articulation assembly (110) relative to selected portions of shaft assembly (140). Knob (134) may include any suitable features to rotate end effector (180), articulation assembly (110), and shaft assembly (140) as would be apparent to one skilled in the art in view of the teachings herein.

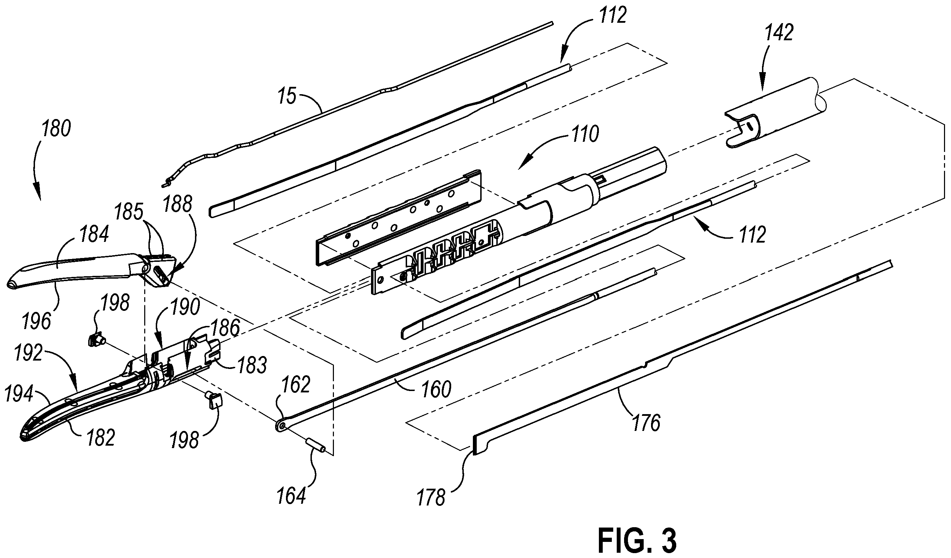

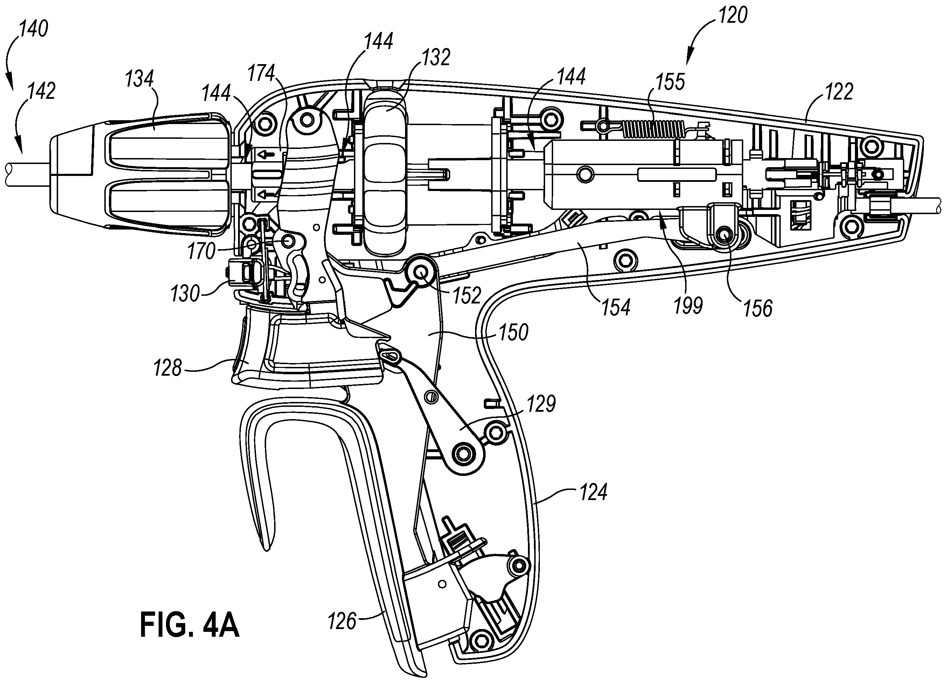

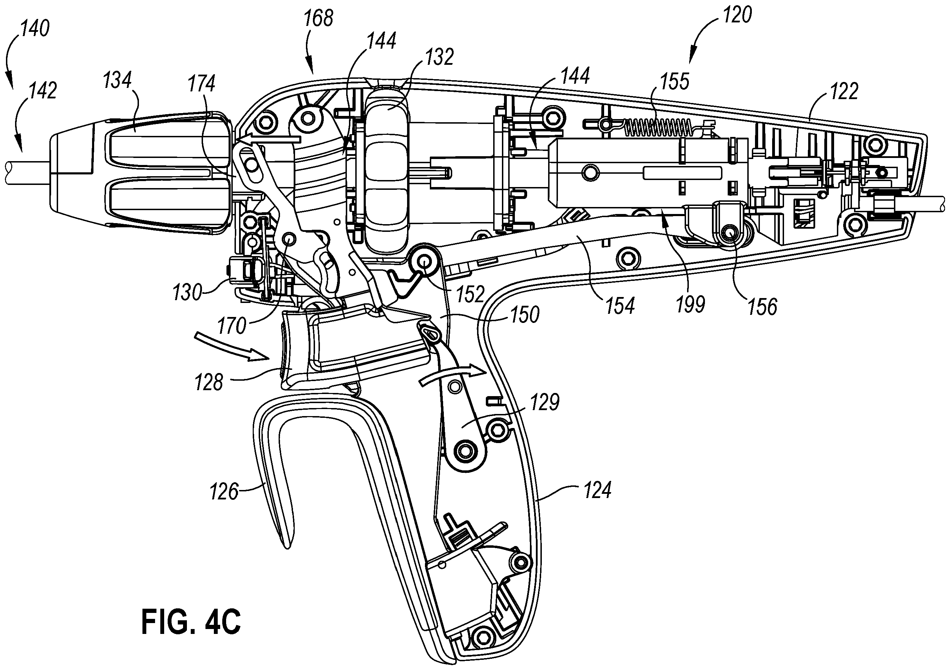

Shaft assembly (140) includes distal portion (142) extending distally from handle assembly (120), and a proximal portion (144) (*see* FIGS. 4A-4B) housed within the confines of body (122) of handle assembly (120). As best shown in FIG. 3, shaft assembly (140) houses a jaw closure connector (160) that couples jaw closure trigger (126) with end effector (180). Additionally, shaft assembly (140) houses a portion of knife member extending between distal cutting edge (178) and knife trigger (128). Shaft assembly (140) also houses actuating members (112) that couple articulation assembly (110) with articulation control (132); as well as an electrical coupling (15) that operatively couples electrode surfaces (194, 196) with activation button (130). As will be described in greater detail below, jaw closure connector (160) is configured to translate relative to shaft assembly (140) to open and close jaws (182, 184) of end effector (180); while knife member (176) is coupled to knife trigger (128) of handle assembly (120) to translate distal cutting edge (178) within the confines of end effector (180); and activation button (130) is configured to activate electrode surface (194, 196).

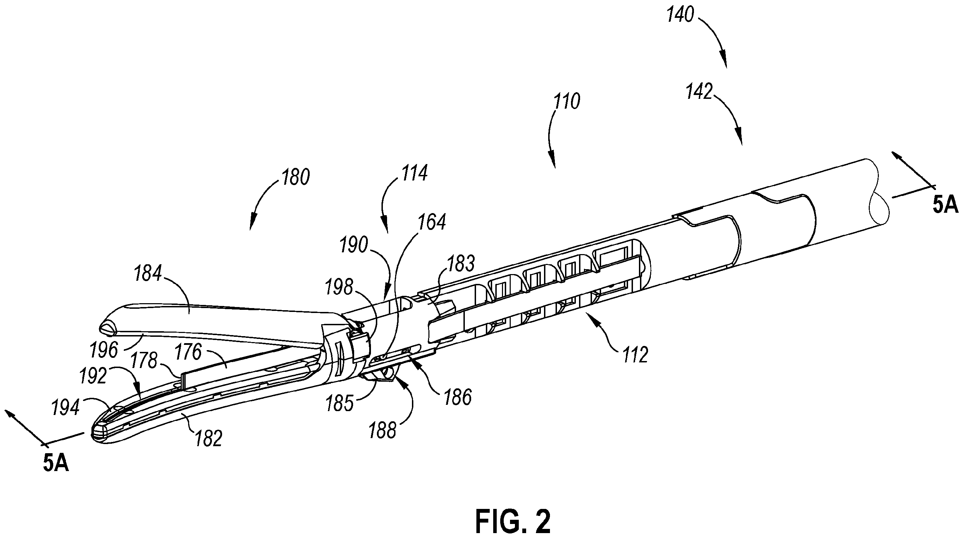

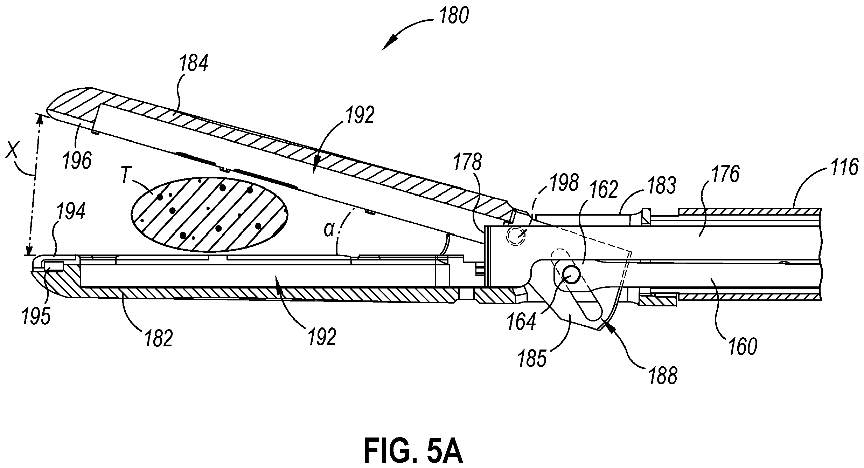

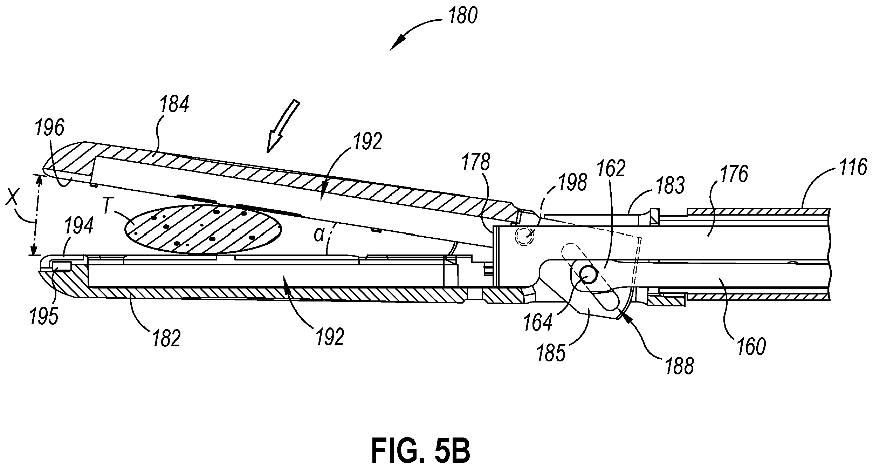

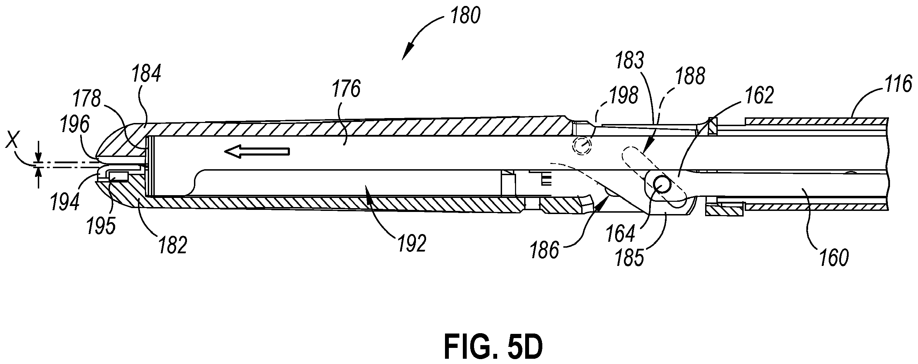

As best seen in FIGS. 2-3, end effector (180) includes lower jaw (182) pivotally coupled with upper jaw (184) via pivot couplings (198). Lower jaw (182) includes a proximal body (183) defining a slot (186), while upper jaw (184) includes proximal arms (185) defining a slot (188). Lower jaw (182) also defines a central channel (190) that is configured to receive proximal arms (185) of upper jaw (184), portions of knife member (176), jaw closure connecter (160), and pin (164). Slots (186, 188) each slidably receive pin (164), which is attached to a distal coupling portion (162) of jaw closure connector (160). Additionally, as best seen in FIGS. 5A-5D, lower jaw (182) includes a force sensor (195) located at a distal tip of lower jaw (182). Force sensor (195) may be in communication with control unit (102). Force sensor (195) may be configured to measure the closure force generated by pivoting jaws (182, 184) into a closed configuration in accordance with the description herein. Additionally, force sensor (195) may communicate this data to control unit (102). For example, force sensor (195) may take the form of a strain gauge.

While in the current example, force sensor (195) is incorporated into instrument (100) and is in communication with control unit (102), other suitable sensors or feedback mechanisms may be incorporated into instrument (100) while in communication with control unit (102) as would be apparent to one skilled in the art in view of the teachings herein. For instance, an articulation sensor or feedback mechanism may be incorporated into instrument (100), where the articulation sensor communicates signals to control unit (102) indicative of the degree end effector (180) is deflected from the longitudinal axis (LA) by articulation control (132) and articulation assembly (110).

As will be described in greater detail below, jaw closure connector (160) is operable to translate within central channel (190) of lower jaw (182). Translation of jaw closure connector (160) drives pin (164). As will also be described in greater detail below, with pin (164) being located within both slots (186, 188), and with slots (186, 188) being angled relative to each other, pin (164) cams against proximal arms (185) to pivot upper jaw (184) toward and away from lower jaw (182) about pivot couplings (198). Therefore, upper jaw (184) is configured to pivot toward and away from lower jaw (182) about pivot couplings (198) to grasp tissue (T). The term "pivot" does not necessarily require rotation about a fixed axis and may include rotation about an axis that moves relative to end effector (180). Therefore, the axis at which upper jaw (184) pivots about lower jaw (182) may translate relative to both upper jaw (184) and lower jaw (182). Any suitable translation of the pivot axis may be used as would be apparent to one skilled in the art in view of the teachings herein. As used herein, jaw distance (X) is the distance between jaws (182, 184) at a distal end portion of jaws (182, 184) and jaw angle (α) is the angle between inner surfaces of jaws (182, 184).

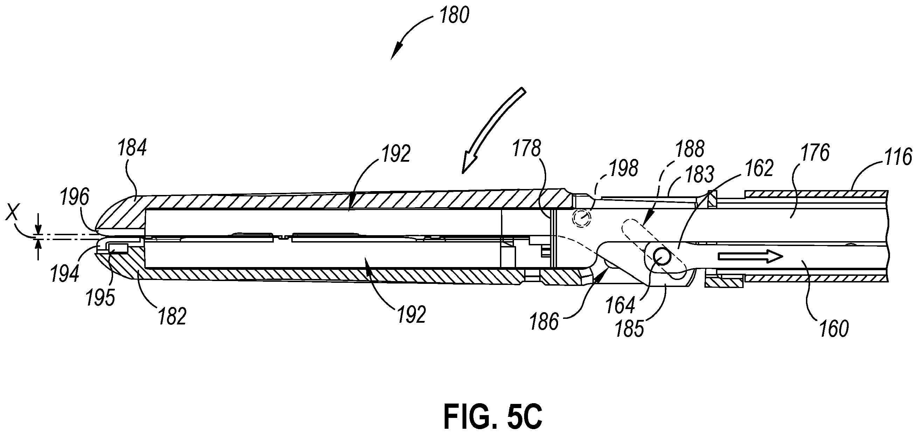

Lower jaw (182) and upper jaw (184) also define a knife pathway (192). Knife pathway (192) is configured to slidably receive knife member (176), such that knife member (176) may be retracted (as shown in FIGS. 5A-5B), and advanced (as shown in FIG. 5C), to cut tissue captured between jaws (182, 184). Lower jaw (182) and upper jaw (184) each comprise a respective electrode surface (194, 196). The power source may provide RF energy to electrode surfaces (194, 196) via electrical coupling (15) that extends through handle assembly (120), shaft assembly (140), articulation assembly (110), and electrically couples with one or both of electrode surfaces (194, 196). Electrical coupling (15) may selectively activate electrode surfaces (194, 196) in response to an operator pressing activation button (130). In some instances, control unit (102) may couple electrical coupling (15) with activation button (130), such that control unit (102) activates electrode surfaces (194, 196) in response to operator pressing activation button (130). Control unit (102) may have any suitable components in order to perform suitable functions as would be apparent to one skilled in the art in view of the teachings herein. For instance, control unit (102) may have a processor, memory unit, suitable circuitry, etc.

FIGS. 4A-5D show an exemplary use of instrument (100) for end effector (180) to grasp, cut, and seal/weld tissue. As described above, and as shown between FIGS. 4A-4B and 5A-5C, jaw closure trigger (126) may be pivoted toward and away from pistol grip (124) and/or body (122) to open and close jaws (182, 184) of end effector (180) to grasp tissue (T). In particular, as will be described in greater detail below, pivoting jaw closure trigger (126) toward pistol grip (124) may proximally actuate jaw closure connector (160) and pin (164), which in turn cams against slots (188) of proximal arms (185) of upper jaw (184), thereby rotating upper jaw (184) about pivot couplings (198) toward lower jaw (182) such that jaws (182, 184) achieve a closed configuration.

Handle assembly (120) further includes a yoke assembly (199) that is slidably coupled along proximal portion (144) of shaft assembly (140). Yoke assembly (199) is operatively coupled with jaw closure connector (160) such that translation of yoke assembly (199) relative to proximal portion (144) of shaft assembly (140) translates jaw closure connector (160) relative to shaft assembly (140). As best seen in FIGS. 4A-4C, yoke assembly (199) is coupled to a body (150) of jaw closure trigger (126) via a link (154). Link (154) is pivotally coupled with yoke assembly (199) via pin (156); while link (154) is also pivotally coupled with body (150) of jaw closure trigger (126) via pin (152). Additionally, jaw closure trigger (126) is pivotally coupled with body (122) of handle assembly (120) via pin (170). Therefore, as shown between FIGS. 4A-4B, an operator may pull jaw closure trigger (126) toward pistol grip (124), thereby rotating jaw closure trigger (126) about pin (170). Rotation of jaw closure trigger (126) leads to rotation of link (154) about both pins (152, 156), which in turn drives yoke assembly (199) in the proximal direction along proximal portion (144) of shaft assembly (140).

As described above, jaw closure connector (160) extends within shaft assembly (140), articulation assembly (110), and central channel (190) of lower jaw (182). As also mentioned above, jaw closure connector (160) is attached to pin (164). Therefore, as seen between FIGS. 5A-5C, proximal translation of yoke assembly (199) leads to proximal translation of pin (164), which in turn cams against slots (188) of proximal arms (185) of upper jaw (184), thereby rotating upper jaw (184) about pivot couplings (198) toward lower jaw (182) such that jaws (182, 184) reduce jaw distance (X) and jaw angle (α) and achieve the closed configuration. As best seen in FIGS. 4A-4C, yoke assembly (199) is also coupled with a bias spring (155). Bias spring (155) is also coupled to a portion of body (122), such that bias spring (155) biases yoke assembly (199) to the position shown in FIG. 4A (associated with the open configuration of end effector (180) as shown in FIG. 5A). Therefore, if an operator releases jaw closure trigger (126), bias spring (155) will translate yoke assembly (199) to the position shown in FIG. 4A, thereby opening jaws (182, 184) of end effector (180).

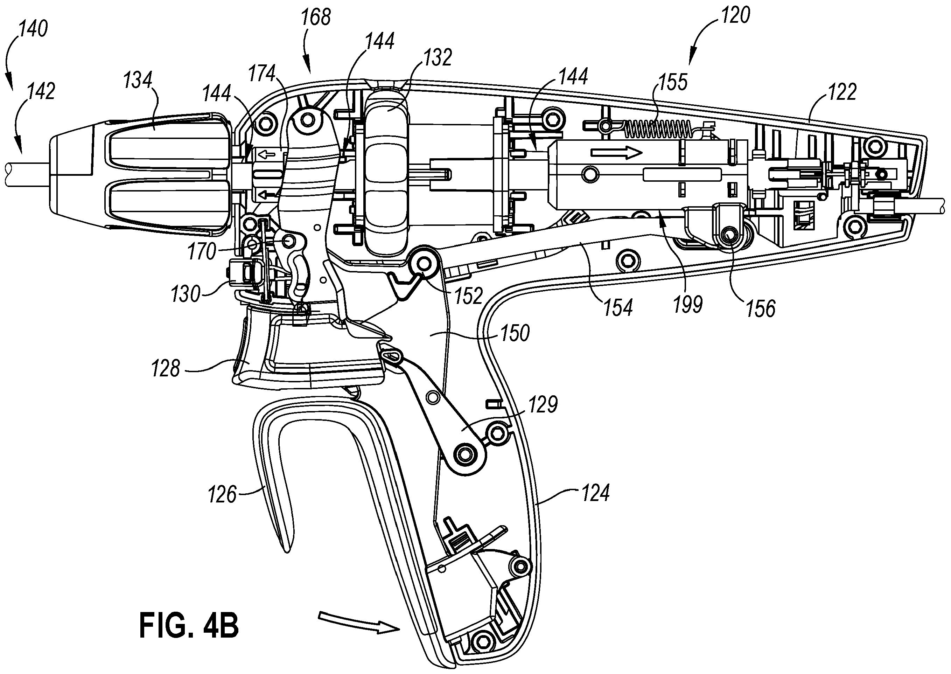

As described above, and as shown between FIGS. 4B-4C and 5C-5D, knife trigger (128) may be pivoted toward and away from body (122) and/or pistol grip (124) to actuate knife member (176) within knife pathway (192) of jaws (182, 184) to cut tissue captured between jaws (182, 184). In particular, handle assembly (120) further includes a knife coupling body (174) that is slidably coupled along proximal portion (144) of shaft assembly (140). Knife coupling body (174) is coupled with knife member (176) such that translation of knife coupling body (174) relative to proximal portion (144) of shaft assembly (140) translates knife member (176) relative to shaft assembly (140).

As best seen in FIGS. 4B-4C and 5C-5D, knife coupling body (174) is coupled a knife actuation assembly (168) such that as knife trigger (128) pivots toward body (122) and/or pistol grip (124), knife actuation assembly (168) drives knife coupling body (174) distally, thereby driving knife member (176) distally within knife pathway (192). Knife trigger (128) is biased to the positions seen in FIGS. 4A-4B (associated with the knife member (176) in the retracted position) by a bias arm (129). With distal cutting edge (178) of knife member (176) actuated to the advance position (position shown in FIG. 5C), an operator may press activation button (130) to selectively activate electrode surfaces (194, 196) of jaws (182, 184) to weld/seal severed tissue that is captured between jaws (182, 184). It should be understood that the operator may also press activation button (130) to selectively activate electrode surfaces (194, 196) of jaws (182, 184) at any suitable time during exemplary use. Therefore, the operator may also press activation button (130) while knife member (176) is retracted as shown in FIGS. 3A-3B. Next, the operator may release jaw closure trigger (126) such that jaws (182, 184) pivot into the opened configuration, releasing tissue.

### II. Exemplary Alternative Electrosurgical Instruments with Clamp Closure Sensors

As previously described with reference to FIGS. 5A-5D, lower jaw (182) includes a force sensor (195) located at a distal tip of lower jaw (182). Force sensor (195) may measure the closure force generated by pivoting jaws (182, 184) into a closed configuration. Additionally, force sensor (195) may be in communication with control unit (102), and communicate this closure force to control unit (102).

While measuring the closure force generated by pivoting jaws (182, 184) of electrosurgical instrument (100) may provide feedback to improve performance of electrosurgical instrument (100), adding sensors (e.g., force sensor (195)) to end effector (180) of electrosurgical instrument (100) may be expensive and/or difficult to dedicate suitable space within electrosurgical instrument (100). For example, it may be expensive and/or difficult to place force sensor (195) and route accompanying wires at distal tip of lower jaw (182) and through end effector (180), shaft assembly (140), and handle assembly (120) to suitably communicate with control unit (102). As a result, it may be desirable to measure closure force to close jaws (182, 184) and/or jaw position (e.g., jaw distance (X) and/or jaw angle (α)) in a low-cost space saving manner.

### A. First Exemplary Alternative Electrosurgical Instrument

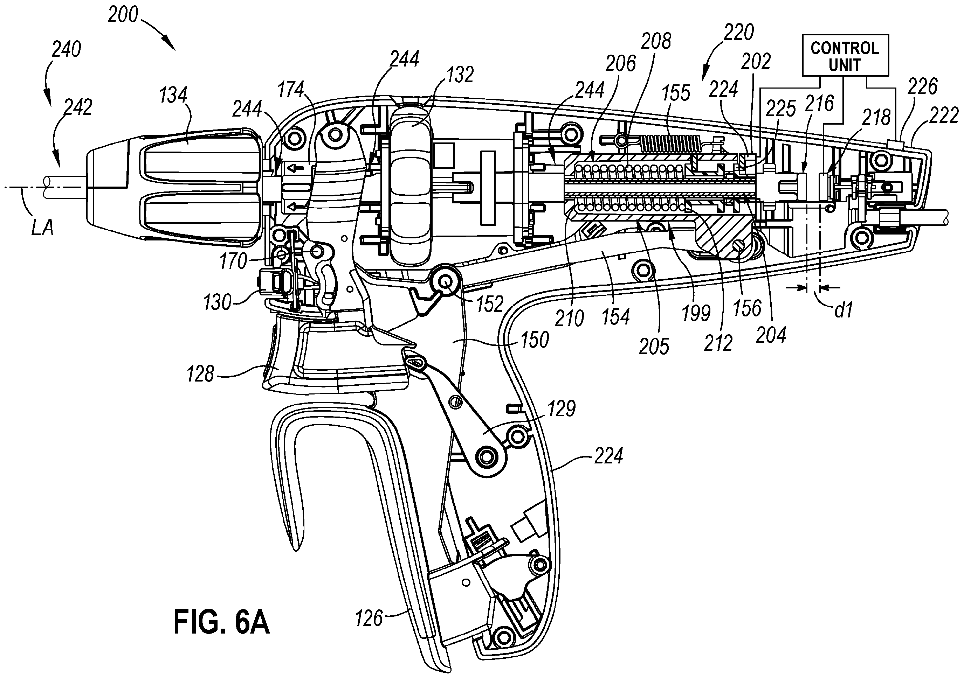

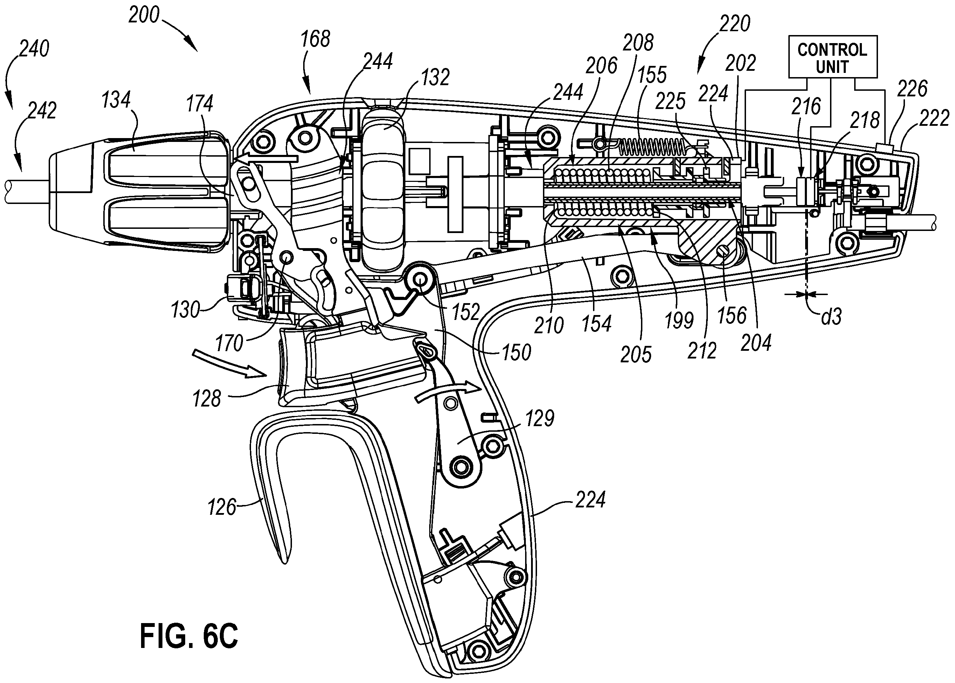

FIGS. 6A-10 show a first exemplary alternative electrosurgical instrument (200), which is similar to electrosurgical instrument (100) shown and described above with reference to FIGS. 1-5D. Electrosurgical instrument (200) includes end effector (180), a controller (shown as control unit (202) that is similar to control unit (102)), a shaft assembly (240) that is similar to shaft assembly (140), and a handle assembly (220) that is similar to handle assembly (120), with differences described below. As will be described in greater detail below, electrosurgical instrument (200) includes first and second clamp closure sensors (218, 224) instead of force sensor (195).

End effector (180) extends distally from shaft assembly (240). At least one of jaws (182, 184) is configured to pivot relative to the other of jaws (182, 184) between an open configuration configured to receive tissue (T) and a closed configuration configured to clamp tissue (T). While FIGS. 5A-5D show upper jaw (184) pivoting relative to lower jaw (182), in other versions, lower jaw (182) may pivot relative to upper jaw (184), or both jaws (182, 184) may pivot, although jaws (182, 184) may alternatively move relative to each other and are not unnecessarily limited to pivoting as discussed in the present example. Electrode assembly (193) may include electrode surfaces (194, 196) which are operable to apply RF energy to tissue (T). Similar to shaft assembly (140), shaft assembly (240) includes distal portion (242) extending distally from handle assembly (220), and a proximal portion (244) housed within the confines of body (122) of handle assembly (220).

Handle assembly (220) is similar to handle assembly (120). As shown, handle assembly (220) includes an optional display (226) configured to indicate to a user whether a tissue load force exceeds a predetermined value corresponding to an incomplete sealing using jaws (182, 184) of the electrosurgical instrument (200). Display (226) is in communication with control unit (202) using either a wired connection or a wireless connection. In some versions, display (226) may include one or more light emitting diodes (LEDs) that may inform the user of different clamping scenarios based on the color of the LED. Other suitable displays are also envisioned.

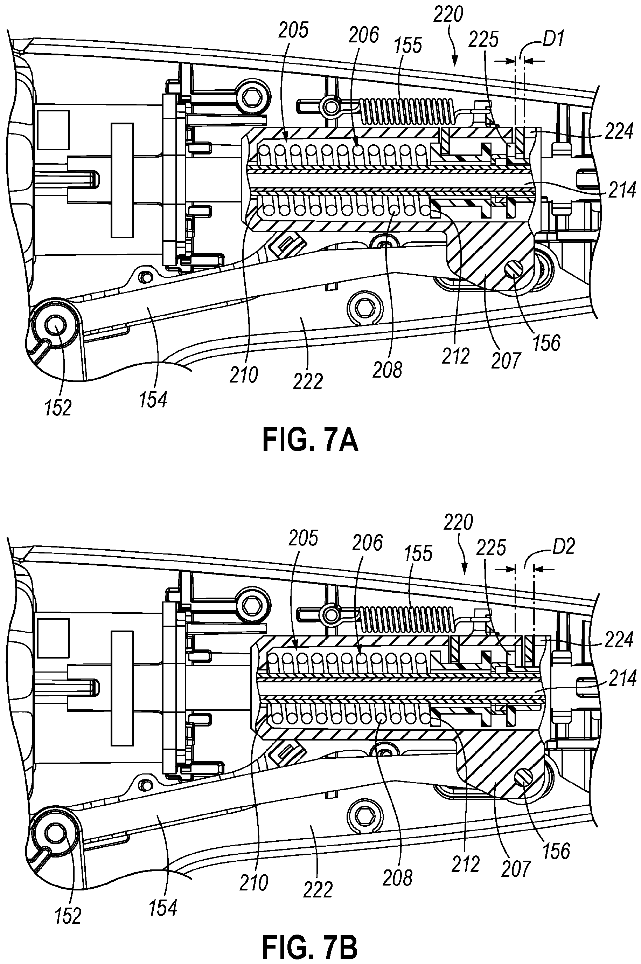

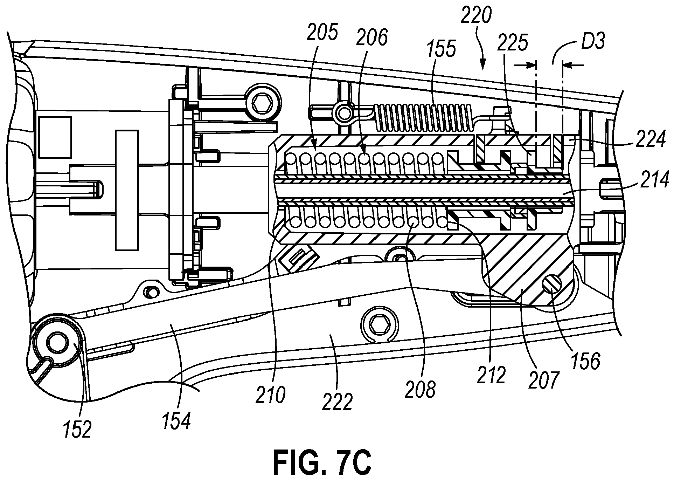

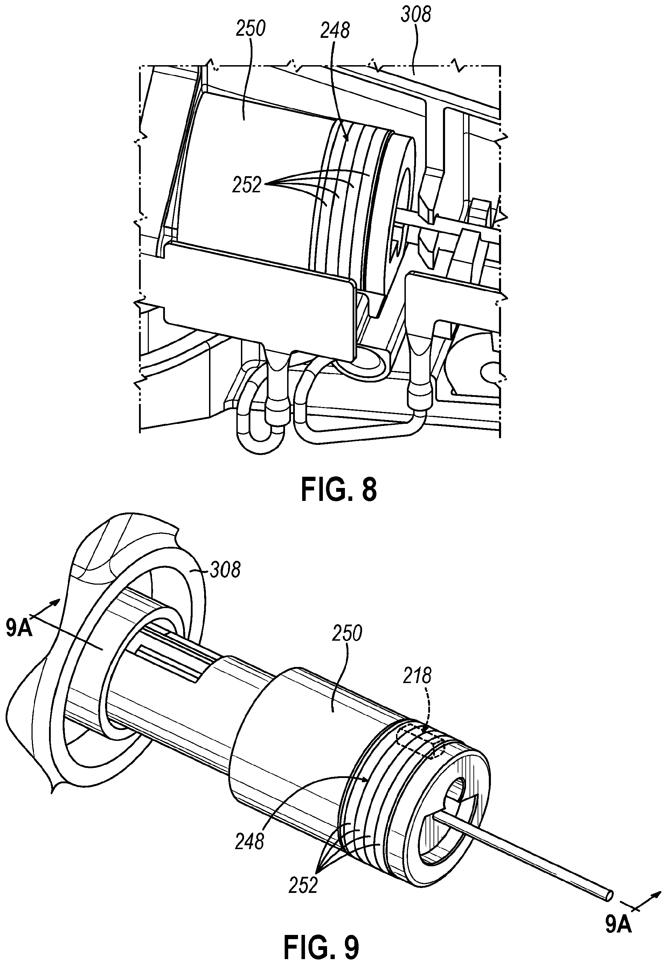

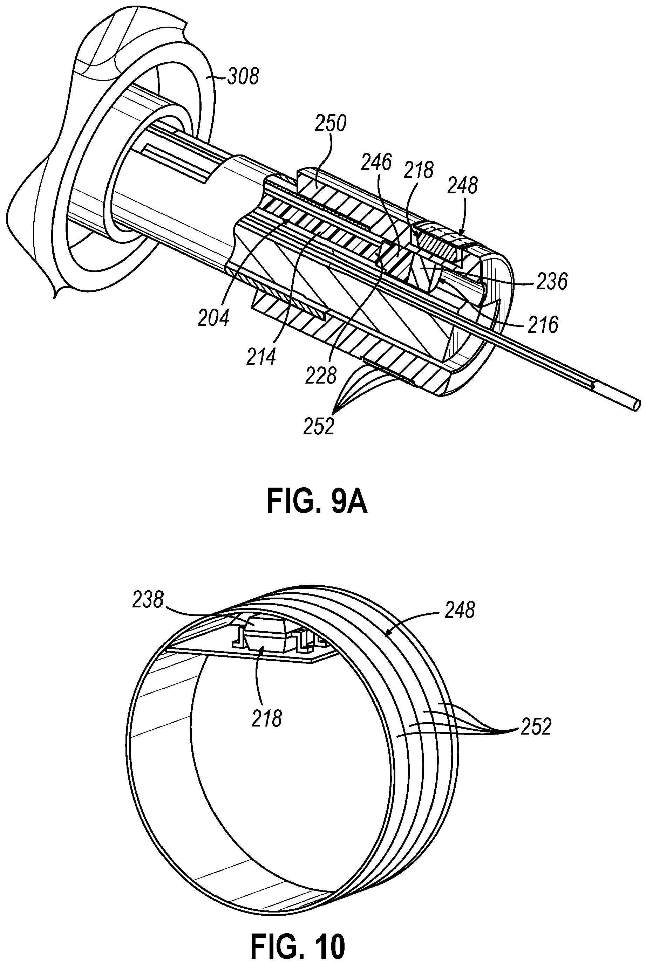

A clamping drive train (204) extends through handle assembly (220) and is configured to move jaws (182, 184) from the open configuration to the closed configuration in response to the actuator being actuated. Clamping drive train (204) is mechanically connected between the actuator and end effector (180). While the actuator is shown as jaw closure trigger (126), which is configured to be manually actuated by a user, other suitable actuators are also envisioned. Clamping drive train (204) includes an actuation rod (214) (*see* FIGS. 7A-7C and 9A) configured to move (e.g., translate) within shaft assembly (to move jaws (182, 184) from the open configuration toward the closed configuration. Actuation rod (214) includes a proximal end (228) (*see* FIG. 9A) and a distal end (not shown). At least a portion of clamping drive train (204) (e.g., jaw closure connector (160)) is configured to translate along longitudinal axis (LA) as jaws (182, 184) move from the open configuration to the closed configuration. Actuation rod (214) is translatably coupled with jaw closure connector (160), and in some versions, may collectively comprise a single component.

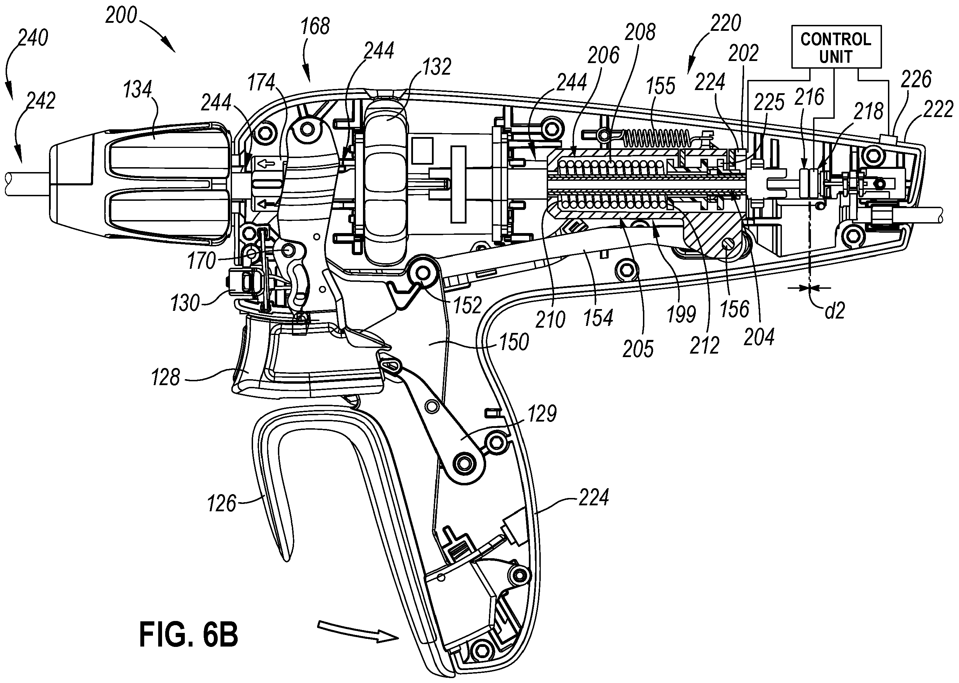

The operation of clamp closure sensor (218) is described with reference to FIGS. 6A-6C. Clamp closure sensor (218) is configured to sense movement of drive train assembly (204). Clamp closure sensor (218) is configured to emit a signal that is influenced (e.g., reflected) by sensor receiver mechanism (216) to generate first sensor information. As shown in FIGS. 6A-6C, the distance between clamp closure sensor (218) and sensor receiver mechanism (216) is shown as distances (d1, d2, d3). In comparing FIGS. 6A and 6B, actuation rod (214) of drive train assembly (204) translates proximally causing distance (d1) to be greater than distance (d2). As shown, the magnitude of distance (d1) is greater than the magnitude of distances (d2, d3). In some versions, distances (d2, d3) may be approximately equal as the actuator is fully actuated. First sensor information may relate to movement of the proximal end (228) of actuation rod (214) as clamping drive train (204) moves jaws (182, 184) from the open configuration toward the closed configuration in response to the actuator being moved from the unactuated position to the actuated position. As shown, clamp closure sensor (218) is disposed proximal to the biasing feature (206). However, clamp closure sensor (218) may be alternately positioned in line with biasing feature (206) or distal to biasing feature (206). An exemplary clamp closure sensor (218) and accompanying sensor receiver mechanism (216) is shown in FIG. 9A.

Clamping drive train (204) includes a portion of yoke assembly (199) that is slidably coupled along proximal portion (244) of shaft assembly (240). Yoke assembly (199) is operatively coupled with jaw closure connector (160), such that translation of yoke assembly (199) relative to proximal portion (244) of shaft assembly (240) translates jaw closure connector (160) relative to shaft assembly (140). Yoke assembly (199) of the present example includes a force limiter (205) operatively connected between jaw closure trigger (126) and upper jaw (184) and configured to limit force transmitted therethrough to a predetermined maximum force for inhibiting overly compressing tissue. In this respect, jaw closure trigger (126) is configured to be selectively moved in a proximal direction from an unactuated position to an actuated position to respectively move upper jaw (184) from the open configuration toward a closed configuration. In the event that reactionary forces against jaws (182, 184) compressing tissue therebetween approach the predetermined maximum force, force limiter (205) allows movement of upper jaw (184) to resiliently slip relative to movement of jaw closure trigger (126) while continuing to apply force to tissue. While force limiter (205) is shown in yoke assembly (199) in the present example, force limiter (205) may generally be positioned between an input actuator (such as jaw closure trigger (126)) and upper jaw (184).

Force limiter (205) includes a biasing feature (206) configured to be biased by a body member (207) of yoke assembly (199) in response to a tissue load being exerted on jaws (182, 184) by tissue (T) to accommodate the above slip. Biasing feature (206) is operatively connected between the actuator and end effector (180) and configured to resiliently deflect for limiting force transmitted therethrough to the tissue. Biasing feature (206) includes a compression spring (208) that includes opposing first and second ends (210, 212). As previously described, jaw closure connector (160) is operable to translate within central channel (190) of lower jaw (182), so that translation of jaw closure connector (160) drives pin (164) that cams against proximal arms (185) to pivot upper jaw (184) toward and away from lower jaw (182) about pivot couplings (198).

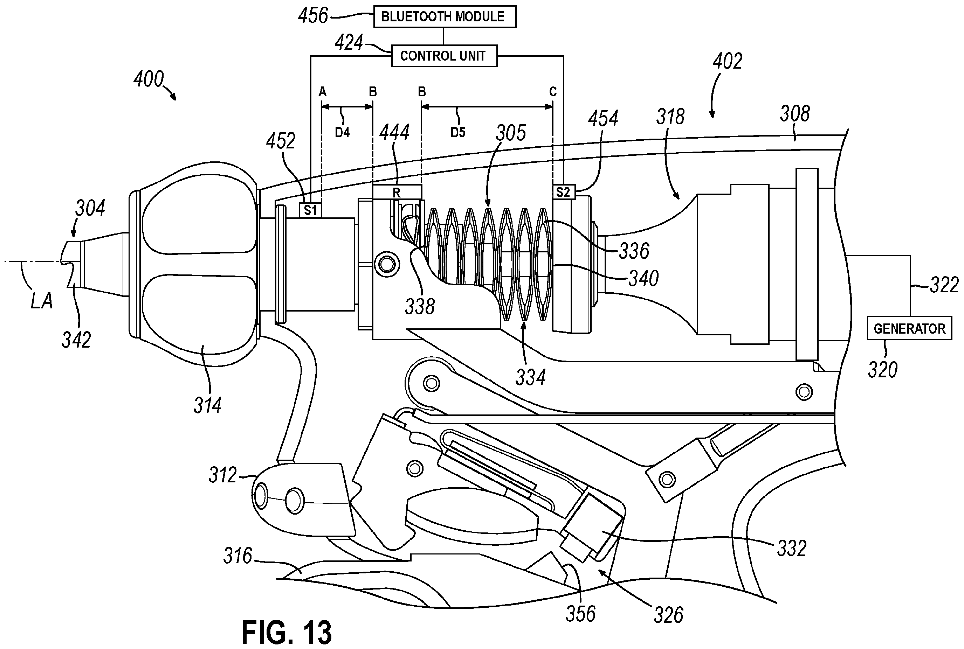

Clamp closure sensor (224) may measure the tissue load force exerted on jaws (182, 184) from within handle assembly (220). Clamp closure sensor (224) is configured to emit a signal that is influenced (e.g., reflected) by sensor receiver mechanism (225) to generate the second sensor information. In other words, the position of sensor receiver mechanism (225) may be sensed by clamp closure sensor (224) to determine the second sensor information. Clamp closure sensor (224) may be configured to produce the second sensor information relating to movement of biasing feature (206) that corresponds force on end effector (180). In some versions, clamp closure sensor (224) may include at least one of a Hall Effect sensor, a light sensor, a resistive sensor, a capacitive sensor, or a linear variable differential transformer. In some versions, clamp closure sensors (218, 224) include first and second reflective optocouplers as described below with reference to FIG. 13. Other suitable clamp closure sensors (224) are also envisioned. Clamp closure sensor (224) may be coupled with handle assembly (220). Particularly in FIGS. 6A-7C, clamp closure sensor (224) is shown as being coupled to yoke assembly (199) within handle assembly (220).

Biasing feature (206) may function as a load cell. Yoke assembly (199) includes a compression spring (208) between jaw closure trigger (126) and clamping drive train (204). Knowing the distance compression spring (208) compresses when jaw closure trigger (126) is transitioned to a fully clamped state provides information as to the compression load jaws (182, 184) deliver to tissue (T). The less clamping drive train (204) moves, the more compression spring (208) compresses resulting in greater force being delivered to clamping drive train (204). The more clamping drive train (204) moves, the less compression spring (208) is compressed resulting in less compression force being delivered to tissue (T). Since the spring constant (k value) of a particular compression spring (208) is known, changes in length of compression spring (208) allow for compression force to be assessed. The force (F) exerted by compression spring (208) on objects attached to its ends is proportional to the spring's change in length away from its equilibrium length and is directed towards the spring's equilibrium position, and is given by the following equation F=-kx. When compression spring (208) is stretched or compressed, length of compression spring (208) changes by an amount (x) from the equilibrium length, then the spring exerts a force (F = -kx) in a direction towards its equilibrium position. The spring constant (k) may be affected by various parameters including the total length of spring (a shorter spring produces a stiffer spring), the thickness of spring wire (a spring using a thicker gauge wire produces a stiffer spring), and the diameter of coils of the spring (a spring with smaller diameter coils produces a stiffer spring). As shown in FIGS. 7A-7C, the distance compression spring (208) compresses corresponds to distances (D1, D2, D3) as jaw closure connector (160) is forced proximally as described above with reference to FIGS. 5A-5D in the present example.

Control unit (202), shown schematically, may include similar structure and functionality as control unit (102) as well as additional structure and/or functionality as described below. Control unit (202) may be disposed within handle assembly (220) or external to handle assembly (220). Control unit (202) is operatively connected with clamp closure sensors (218, 224). If desired, sensor information may be communicated wirelessly to control unit (202). For example, a Bluetooth module (e.g., Bluetooth module (456)) may be used to read the sensor information from clamp closure sensors (218, 224). Control unit (202) is configured to use the sensor information from clamp closure sensors (218, 224) to determine at least one of the tissue load force exerted on end effector (180), or a position of one of jaws (182, 184) relative to the other of one of jaws (182, 184). In some versions, the jaw position includes a jaw angle defined between jaws (182, 184). The sensor information from clamp closure sensor (218) allows for jaw position (e.g., jaw distance (X) and/or jaw angle (α)) to be determined.

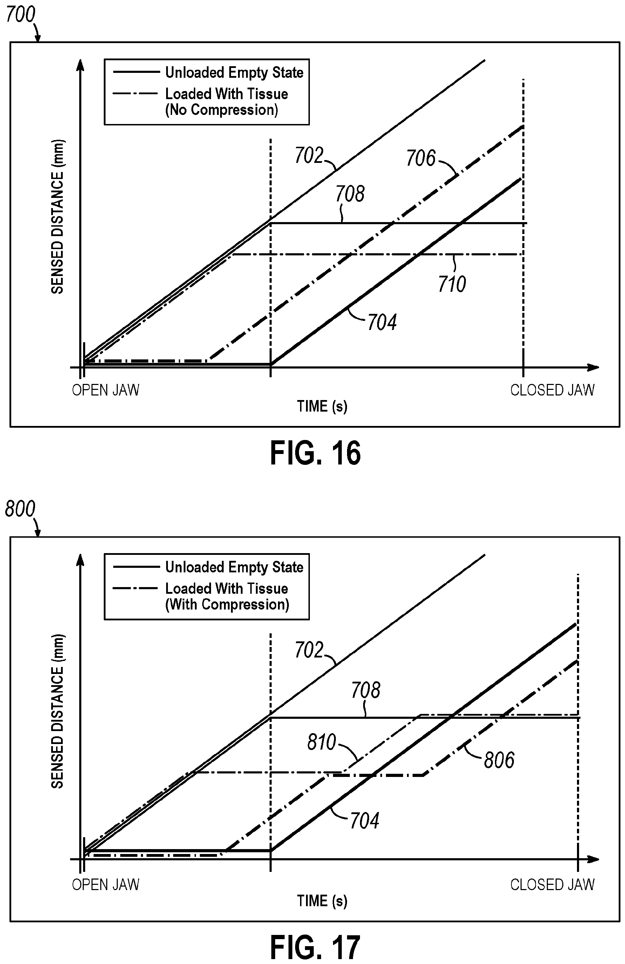

Control unit (202) is configured to use compression of compression spring (208) as sensed using clamp closure sensor (224) to produce second sensor information to indirectly measure the tissue load exerted on end effector (180). For example, control unit (202) is configured to use the information from clamp closure sensor (224) to measure compression of compression spring (208) to determine, such as by estimating, the tissue load exerted on end effector (180) and the position of upper jaw (184). The position of jaws (182, 184) relates to whether fluid has evacuated tissue (T) before sealing tissue (T) using electrode assembly (193). The estimation by control unit (202) is described further in FIG. 16 regarding method (1100).

One of clamp closure sensor (218) or sensor receiver mechanism (216) may translate together with a portion of clamping drive train (204) as jaws (182, 184) move from the open configuration to the closed configuration. For example, one of clamp closure sensor (218) or the first sensor receiver mechanism is coupled with a proximal end (228) of actuation rod (214). As shown, sensor receiver mechanism (216) is translatably coupled with clamping drive train (204). In some versions, sensor receiver mechanism (216) may be directly coupled with clamping drive train (204). Clamp closure sensor (218) includes at least one of an optical reflective sensor, an optical linear encoder, a magnetic sensor, or a capacitive sensor. In some versions, sensor receiver mechanism (216) may include at least one of a magnet or a reflector. However, other suitable clamp closure sensors and/or sensor receiver mechanisms (216) are also envisioned. As shown in FIG. 9A, sensor receiver mechanism (216) includes a magnet (236), and clamp closure sensor (218) includes a Hall Effect sensor (238) configured to sense magnet (236). An intermediate coupling magnet (246) may be disposed between proximal end (228) of actuation rod (214) and magnet (236). Intermediate coupling magnet (236) may be rigidly coupled with proximal end (228) of actuation rod (214) using at least one of an adhesive or a press fit connection. Use of intermediate coupling magnet (246) with magnet (236) may reduce misalignments between components.

Clamp closure sensor (218) may operate in conjunction with a slip ring (248). Slip ring (248) is in electrical communication with clamp closure sensor (218). Slip ring (248) is configured to allow for rotation of clamp closure sensor (218) or first sensor receiver mechanism (216) relative to the other of clamp closure sensor (218) or sensor receiver mechanism (216). Slip ring (248) may be formed around a body portion (250), which is shown as being annular. Slip ring (248) includes a plurality of annular spaced contacts (252).

Use of clamp closure sensors (218, 224) may provide one or more benefits to electrosurgical instrument (200). Using clamp closure sensors (218, 224) instead of force sensor (195) may be more cost effective in some instances, as clamp closure sensors (218, 224) are shown as being disposed in handle assembly (220) which prevents routing one or more wires for force sensor (195) disposed in end effector (180). Clamp closure sensors (218, 224) may incorporate inexpensive and/or compact sensors that provide sensor information pertaining to tissue load and jaw position at the same time. The combination of measuring tissue load and jaw position (e.g., jaw distance (X) and/or jaw angle (α)) may provide real time information as to the movement of jaws (182, 184) without directly measuring tissue load and jaw position at end effector (180). For example, clamp closure sensors (218, 224) provide a straightforward method for estimating jaw pressure and jaw position, while also occupying a reduced space within electrosurgical instrument (200).

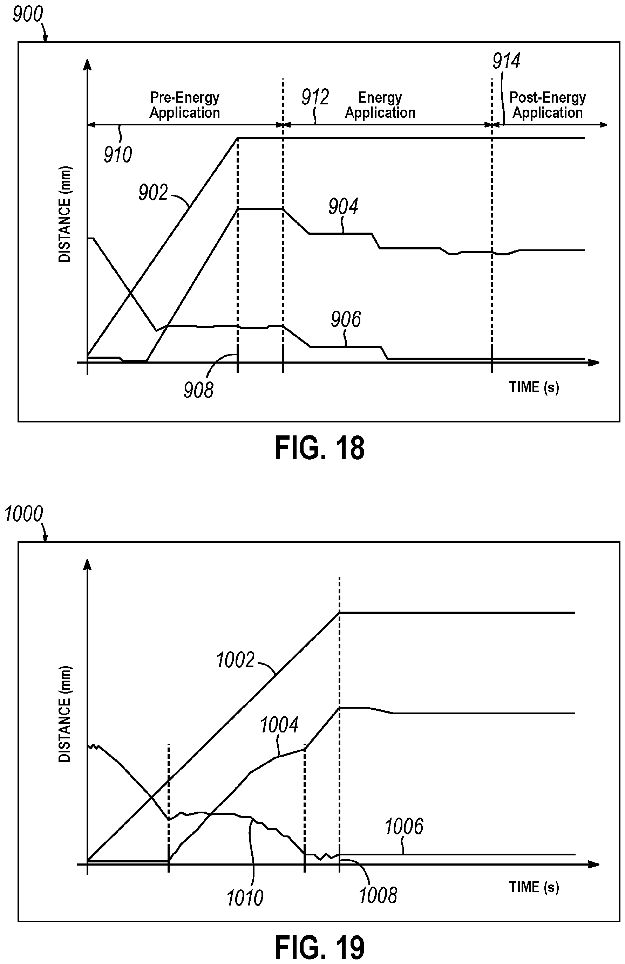

The position of jaws (182, 184) may provide feedback as to when tissue (T) is sealing, since jaws (182, 184) will further close when tissue (T) reaches a sealing temperature sufficient for denaturing as shown and described below with reference to FIG. 18. The position of jaws (182, 184) may also be used to determine when fluid has been sufficiently evacuated from tissue (T) before sealing due to upper jaw (184) slowly dropping while the fluid leaves. Reduced fluid in tissue (T) may reduce heat exchange, which may allow the vessels in tissue (T) to increase to the sealing temperature more reliably. The jaw load may assess tension on tissue (T) in the plane of opening jaws (182, 184). The jaw load may relax over time before sealing and may be indicative of tissue stiffness. The force and position of jaws (182, 184) may indicate that tissue is slipping out from between jaws (182, 184) as shown and described below with reference to FIG. 19.

### B. Second Exemplary Alternative Electrosurgical Instrument

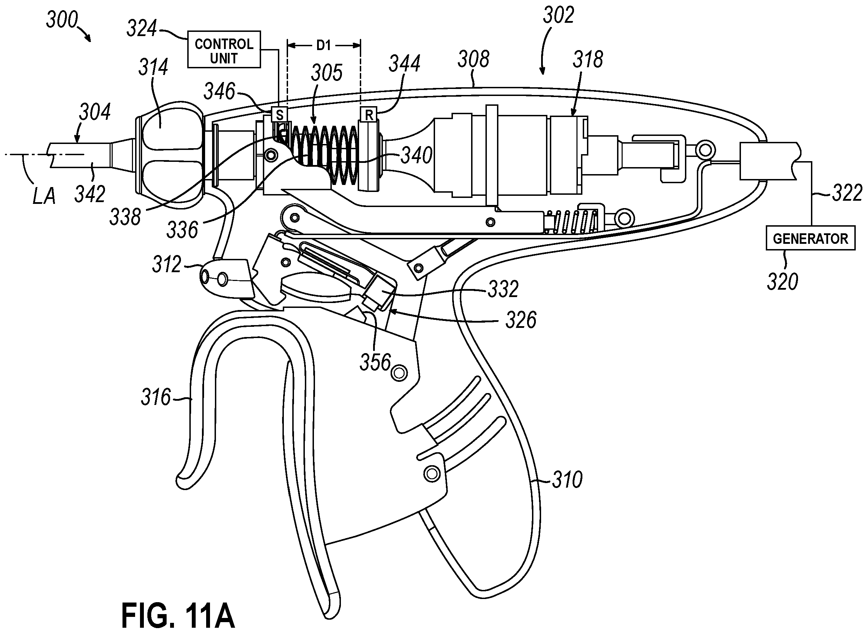

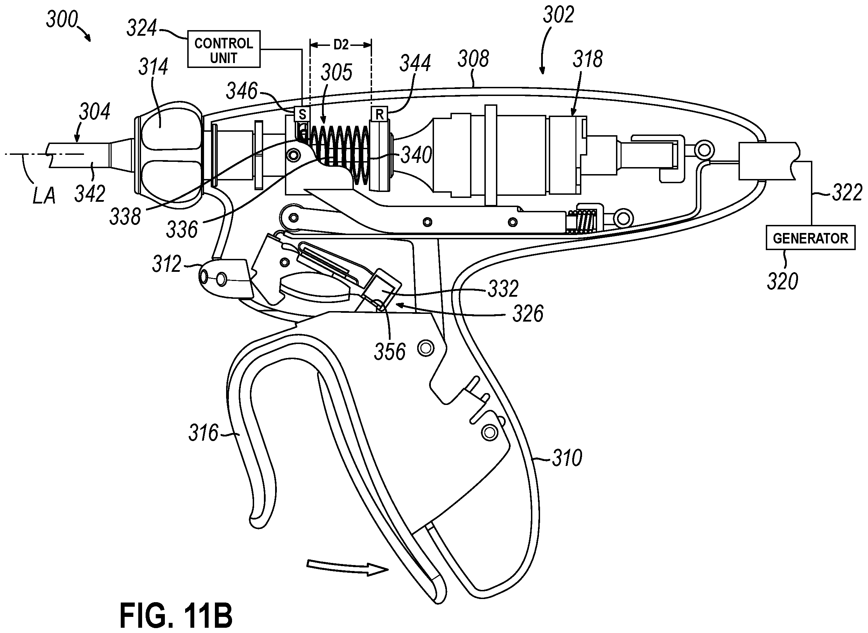

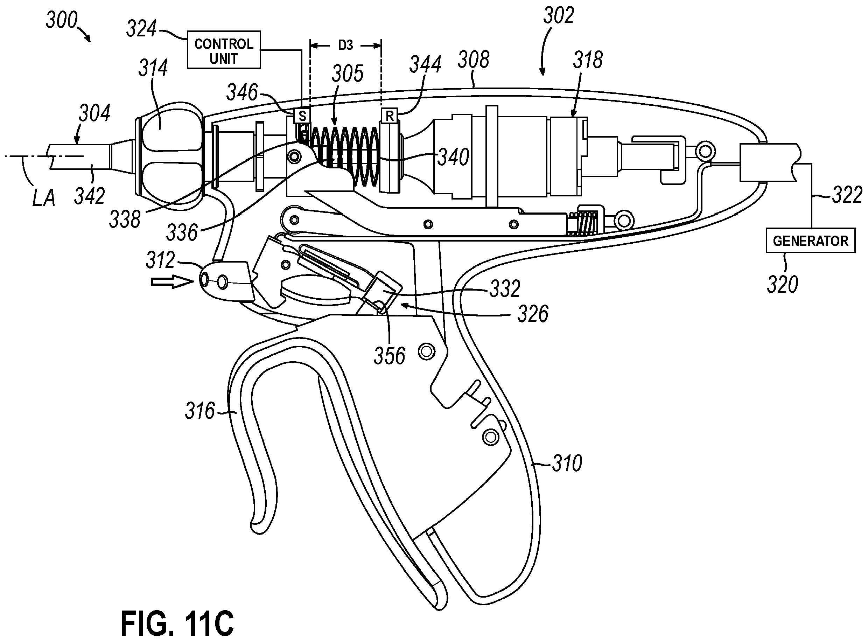

FIGS. 11A-12C show a second exemplary alternative electrosurgical instrument (300) in the form of an ultrasonic surgical instrument. Electrosurgical instrument (300) is operable to cut tissue and seal or weld tissue (e.g., a blood vessel, etc.) substantially simultaneously. Electrosurgical instrument (300) includes a handle assembly (302), a shaft assembly (304), and an end effector (306). Electrosurgical instrument (300) may include aspects in accordance with at least some of the teachings of U.S. Pat. No. 11,051,841, entitled "Ultrasonic Blade and Clamp Arm Matching Design, published July 6, 2021 and/or U.S. Pat. Pub. No. 2020/0345390, entitled "Mechanical Lockout for Ultrasonic Surgical Instrument," published November 5, 2020, the disclosure of each of which is incorporated by reference herein. Handle assembly (302) comprises a body (308) including a pistol grip (310) and one or more buttons (312). Button (312) may provide the operator with varied control of electrosurgical instrument (300). In addition, or in the alternative, button (312) may provide functionality in accordance with at least some of the teachings of U.S. No. 9,949,785, entitled "Ultrasonic Surgical Instrument with Electrosurgical Feature," issued April 24, 2018, the disclosure of which is incorporated by reference herein. Handle assembly (302) also includes a trigger (316) that is pivotable toward and away from pistol grip (310). Handle assembly includes a knob (314) configured to be rotated by a user to rotate end effector (306).

As shown, an ultrasonic transducer assembly (318) extends proximally from body (308) of handle assembly (302). Ultrasonic transducer assembly (318) is integrated within body (308), while in other versions, ultrasonic transducer assembly (318) is removable from body (308). Ultrasonic transducer assembly (318) is coupled with a generator (320) via a cable (322). Ultrasonic transducer assembly (318) receives electrical power from generator (320) and converts that electrical power into ultrasonic vibrations through piezoelectric principles as is known in the art. While not shown, generator (320) cooperates with a controller (shown schematically as control unit (324)) to provide a power profile to transducer assembly (318) that is particularly suited for the generation of ultrasonic vibrations through transducer assembly (318). In addition, or in the alternative, generator (320) may be constructed in accordance with at least some of the teachings of U.S. Pat. No. 8,986,302, entitled "Surgical Generator for Ultrasonic and Electrosurgical Devices," issued March 24, 2015, the disclosure of which is incorporated by reference herein.

Handle assembly (302) includes an end of stroke feature (326) configured to determine that jaw closure trigger (126) is in the actuated position. For example, end of stroke feature (326) may be used of in place of clamp closure sensor (218). End of stroke feature (326) may communicate with control unit (324) (shown schematically) to communicate that the end of the closure stroke has been reached. For example, end of stroke feature (326) includes a switch (332) that is configured to complete a circuit with jaw closure trigger (126) in the actuated position. Switch (332) is configured to contact a contact portion (356) of jaw closure trigger (126) when jaw closure trigger (126) is in the actuated position, which in the present example is a fully actuated position that, without slip, would otherwise move upper jaw (184) to the fully closed configuration. Switch (332) may be positioned in and/or fixed within pistol grip (124); however, other suitable positions of end of stroke feature (326) are also envisioned. In some versions, end of stroke feature (326) may include a sensor (not shown).

Similar to handle assembly (220), handle assembly (302) includes a force limiter (305) operatively connected between trigger (316) and upper jaw (330) and configured to limit force transmitted therethrough to a predetermined maximum force for inhibiting overly compressing tissue. In this respect, trigger (316) is configured to be selectively moved in a proximal direction from an unactuated position to an actuated position to respectively move upper jaw (330) from the open configuration toward a closed configuration. In the event that reactionary forces against jaws (328, 330) compressing tissue therebetween approach the predetermined maximum force, force limiter (305) allows movement of upper jaw (330) to resiliently slip relative to movement of trigger (316) while continuing to apply force to tissue. In addition, switch (332) discussed above is positioned to detect that trigger (316) is in the actuated position, which in the present example is a fully actuated position that, without slip, would otherwise move upper jaw (330) to the fully closed configuration. While force limiter (305) is positioned as shown in the present example, force limiter (305) may generally be positioned between an input actuator, such as trigger (316), and upper jaw (330).

More particularly, force limiter (305) includes a biasing feature (334) configured to be biased in response to a tissue load being exerted on jaws (328, 330) by tissue (T). Biasing feature (334) includes a wave spring assembly (336) having opposing first and second ends (338, 340). At least a portion of clamping drive train (342) is configured to translate proximally along longitudinal axis (LA) as jaws (328, 330) move from the open configuration to the closed configuration. A sensor receiver mechanism (344) may translate together with a portion of clamping drive train (204) as jaws (328, 330) move between the open configuration to the closed configuration. Sensor receiver mechanism (344) may be translatably coupled with clamping drive train (342). In some versions, sensor receiver mechanism (344) may be directly coupled with clamping drive train (342). In some versions, sensor receiver mechanism (344) may include at least one of a magnet or a reflector. However, other suitable sensor receiver mechanisms (344) are also envisioned.

Clamp closure sensor (346) is configured to produce clamp closure sensor information relating to movement of biasing feature (334) in response to the tissue load. Clamp closure sensor (346) may include at least one of a Hall Effect sensor, a light sensor, a resistive sensor, a capacitive sensor, or a linear variable differential transformer. In some versions, a single clamp closure sensor (346) may be used for determining both tissue load exerted on jaws (328, 330) as well as jaw position. As used herein, jaw distance (X) is the distance between jaws (328, 330) and jaw angle (α) is the angle between jaws (328, 330). Other suitable clamp closure sensors (346) are also envisioned. Clamp closure sensor (346) is coupled with handle assembly (302). Particularly, clamp closure sensor (346) is shown as being coupled with an inner surface of body (308) of handle assembly (220). In some versions, clamp closure sensor (346) may be adhered to the inner surface of body (308) using an adhesive. The distance between clamp closure sensor (346) and sensor receiver mechanism (344) is shown as distance (D1, D2, D3). As shown in FIGS. 11A-11C, the distance wave spring assembly (336) compresses corresponds to the magnitude that distance (D1, D2, D3) increases as at least a portion of clamping drive train (342) (shown schematically) is forced proximally.

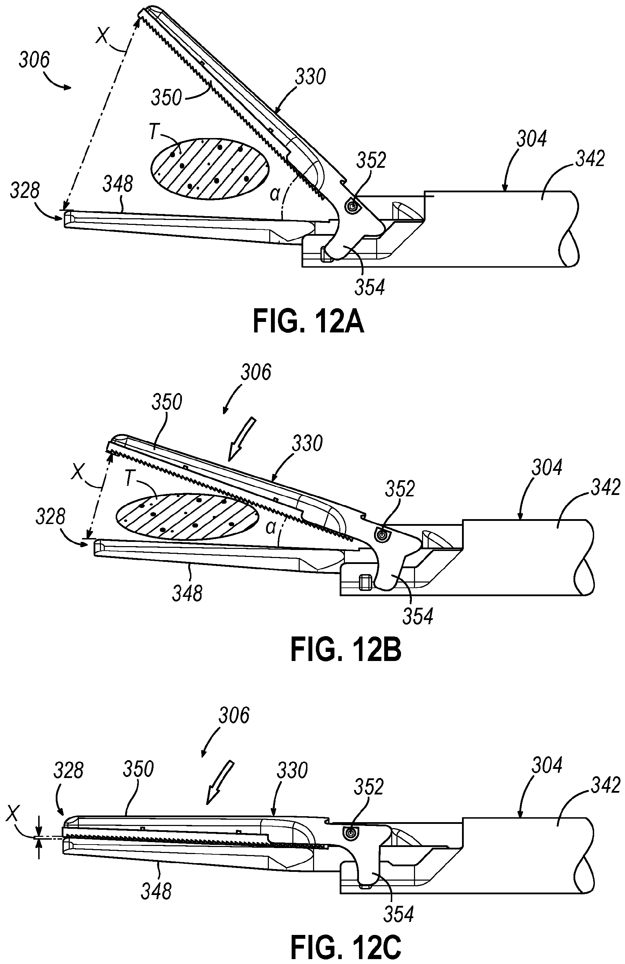

End effector (306) is shown and described with refence to FIGS. 12A-12C. End effector (306) includes an ultrasonic blade (348) and a clamp arm (350) that pivots relative to ultrasonic blade (348). Clamp arm (350) is coupled with trigger (316) such that clamp arm (350) is pivotable toward ultrasonic blade (348) in response to pivoting of trigger (316) toward pistol grip (310); and such that clamp arm (350) is pivotable away from ultrasonic blade (348) in response to pivoting of trigger (316) away from pistol grip (310). Clamp arm (350) is pivotable relative to ultrasonic blade (348) using pivot points (352, 354). Various suitable ways in which clamp arm (350) may be coupled with trigger (316) are disclosed in various patent references cited herein; and further suitable ways in which clamp arm (350) may be coupled with trigger (316) will be apparent to those of ordinary skill in the art in view of the teachings herein.

Ultrasonic blade (348) is positioned at the distal end of an acoustic drivetrain that includes an acoustic waveguide (not shown) and ultrasonic transducer assembly (318) to vibrate ultrasonic blade (348). Ultrasonic blade (348) of the present example is operable to vibrate at ultrasonic frequencies to effectively cut through and seal tissue, particularly when tissue (T) is being clamped between clamp arm (350) and ultrasonic blade (348). When ultrasonic transducer assembly (318) is activated, these mechanical oscillations are transmitted through waveguides to reach ultrasonic blade (348), thereby providing oscillation of ultrasonic blade (348) at the resonant ultrasonic frequency. Thus, when tissue is secured between ultrasonic blade (348) and clamp arm (350), the ultrasonic oscillation of ultrasonic blade (348) may simultaneously sever the tissue and denature the proteins in adjacent tissue cells, thereby providing a coagulative effect with relatively little thermal spread. In some versions, an electrical current may also be provided through ultrasonic blade (348) and clamp arm (350) to also cauterize the tissue. For example, ultrasonic blade (348) and clamp arm (350) may be configured to apply radiofrequency (RF) electrosurgical energy to tissue in addition to being configured to apply ultrasonic energy to tissue.

Control unit (324) is configured to use sensor information from clamp closure sensor (346) to measure compression of wave spring assembly (336) to determine, such as by estimating, the tissue load exerted on end effector (306) and jaw position (e.g., jaw distance (X) and/or jaw angle (α)). The relative position of jaws (328, 330) defines jaw distance (X) and jaw angle (α) between jaws (328, 330). For example, control unit (324) is configured to use the information from clamp closure sensor (346) to measure compression of wave spring assembly (336) to estimate the tissue load exerted on end effector (306) and the position of jaws (328, 330) (e.g., upper jaw (328)). The jaw position (jaw distance (X) and/or jaw angle (α)) may correspond to whether fluid has evacuated tissue (T) before sealing tissue (T) using the energized feature (e.g., electrode assembly (193) and/or ultrasonic blade (348)). This estimation by control unit (202, 324) may use a lookup table or interpolation of compression of wave spring assembly (336).

Use of clamp closure sensor (346) may provide one or more benefits to electrosurgical instrument (300). For example, clamp closure sensor (346) may provide a straightforward method for estimating pressure and jaw position, while also not occupying significant space within electrosurgical instrument (300). Sensor receiver mechanism (344) is configured to be sensed by clamp closure sensor (346) to determine the sensor information. The position of jaws (328, 330) may provide feedback as to when tissue (T) is sealing, since jaws (328, 330) will close further when tissue (T) is reached a sealing temperature sufficient for denaturing. The position of jaws (328, 330) may also be used to determine when a predetermined amount of fluid has been evacuated from tissue (T) before sealing due to upper jaw (328) slowly dropping while the fluid leaves. Less fluid in tissue (T) may reduce heat exchange, which may allow the vessels in tissue (T) to increase to the sealing temperature more reliably. Jaw load may judge tension on tissue (T) in the plane of opening jaws (328, 330). The jaw load relaxing over time before sealing may detect tissue stiffness.

### C. Third Exemplary Alternative Electrosurgical Instrument

FIG. 13 shows a third exemplary alternative electrosurgical instrument (400) in the form of an ultrasonic surgical instrument. Electrosurgical instrument (400) is similar to electrosurgical instrument (300) described in detail above. Similar to electrosurgical instrument (300), electrosurgical instrument (400) includes a handle assembly (402).

Instead of handle assembly (302) including clamp closure sensor (346), handle assembly (402) includes first and second clamp closure sensors (452, 454). As shown, clamp closure sensors (452, 454) are in the form of first and second reflective optocouplers; however, other suitable clamp closure sensors are also envisioned. As shown in FIG. 10, clamp closure sensor (452) measures a distance (D4) between points A and B using a sensor receiver mechanism (444). Similarly, clamp closure sensor (454) measures a distance (D5) between points B and C using sensor receiver mechanism (444). As a result, clamp closure sensor (452) may measure the movement (e.g., translation) of clamping drive train (342) which may estimate the relative position of jaws (328, 330). Similarly, clamp closure sensor (454) may measure compression of wave spring assembly (336) to estimate the force to close jaws (328, 330). Clamp closure sensors (452, 454) may be small and/or compact in size. In some versions, clamp closure sensors (452, 454) are about 2 millimeters by 3 millimeters. Clamp closure sensors (452, 454) may be in communication with a control unit (424) that may include a Bluetooth module (456). Bluetooth module (456) may sample information from clamp closure sensors (452, 454). Bluetooth module (456) may be small in size. In some versions, Bluetooth module (456) may be about 15 millimeters by 20 millimeters.

Sensor receiver mechanism (444) may translate together with a portion of clamping drive train (204) as jaws (328, 330) move between the open configuration and the closed configuration. Sensor receiver mechanism (444) may be translatably coupled with clamping drive train (342). In some versions, sensor receiver mechanism (444) may be directly coupled with clamping drive train (342). In some versions, sensor receiver mechanism (444) may include at least one of a magnet or a reflector. However, other suitable sensor receiver mechanisms (444) are also envisioned.

### D. Exemplary Graphs

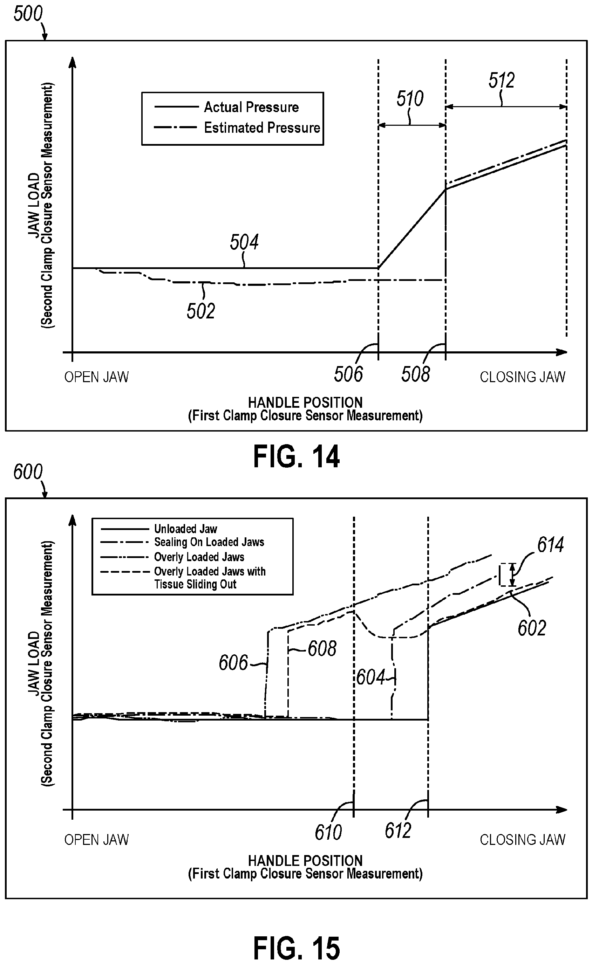

FIG. 14 shows an exemplary graph (500) that includes plots (502, 504) of the position of handle assembly (402) relative to jaw load for electrosurgical instrument (400). Plot (502) relates to expected pressure experienced by closing of jaws (328, 330) of electrosurgical instrument (400). Plot (504) relates to a determined, such as an estimated, pressure experienced by closing of jaws (328, 330) of electrosurgical instrument (400) that may be indirectly measured using clamp closure sensors (452, 454) and sensor receiver mechanism (444). Line (506) corresponds to jaws (328, 330) of electrosurgical instrument (400) being closed in an unloaded empty state (e.g., without tissue (T) being disposed between jaws (328, 330)). Line (508) corresponds to where wave spring assembly (336) starts to compress. Zone (510) refers to a plastic/shaft elastic zone where plot (504) does not track plot (502) because wave spring assembly (336) has not yet started to compress. However, zone (512) refers to a spring compression zone where plot (504) tracks plot (502) because wave spring assembly (336) has not yet started to compress.

FIG. 15 depicts an exemplary graph (600) that includes plots (602, 604, 606, 608) of the position of handle assembly (402) relative to jaw load for various loading conditions for electrosurgical instrument (400). Line (610) corresponds to a limit in distance (X) between jaws (328, 330) of electrosurgical instrument (400) for sealing. Line (612) corresponds to jaws (328, 330) of electrosurgical instrument (400) being closed in an unloaded empty state (e.g., without tissue (T) being disposed between jaws (328, 330)). Plot (602) uses a solid line to refer to closing of jaws (328, 330) of electrosurgical instrument (400) in an unloaded empty state (e.g., without tissue (T) being disposed between jaws (328, 330)). Plot (602) may be used as reference data and compared to plots (604, 606, 608). Plot (604) uses a dash dot dash line to refer to jaws (328, 330) sealing on tissue of a regular thickness. Height (614) at the end of the plot (604) may estimate movement during sealing. While still under the spring force, jaws (328, 330) collapse further such that this movement of jaw (328, 330) is then detected by clamp closure sensors (452, 454). Plot (606) uses a dash dot dot dash line to refer to overly loaded jaws (e.g., overstuffed jaws). Wave spring assembly (336) starts compression with jaws (328, 330) open. Plot (608) uses evenly spaced dashed lines to refer to overly loaded jaws (e.g., overstuffed jaws) where tissue inadvertently slides out of jaws (328, 330). Plot (608) starts like plot (606) but during the closure of jaws (328, 330) the compression decays, which may indicate tissue (T) is getting pushed out from between jaws (328, 330).

FIG. 16 shows an exemplary graph (700) that includes plots (702, 704, 706, 708, 710). Graph (700) shows jaws (182, 184, 328, 330) moving from the open configuration to the closed configuration. Plot (702) corresponds to the position of handle assembly (220, 402), which may be determined by clamp closure sensors (218) sensing clamping drive train (204, 342). Plot (704) corresponds to the compression of biasing feature (206, 334) in the unloaded empty state. Plot (706) corresponds to the compression of biasing feature (206, 334) in a loaded state. Plot (708) refers to the distance between jaws (182, 184) or the distance between jaws (328, 330) in an unloaded state. Plot (710) refers to the distance between jaws (182, 184) or the distance between jaws (182, 184, 328, 330) in the loaded state. Plot (708) may be determined by subtracting plot (706) corresponding to the sensed compression of biasing feature (206, 334) from plot (702) corresponding to the sensed position of clamping drive train (204, 342). Similarly, plot (710) may be determined by subtracting plot (706) corresponding to the sensed compression of biasing feature (206, 334) from plot (702) corresponding to the sensed position of clamping drive train (204, 342). As a result, obtaining sensor information for plot (702) and plot (704) allow for estimation of plot (708). Similarly, obtaining sensor information for plot (702) and plot (706) allow for estimation of plot (710).

FIG. 17 shows an exemplary graph (800) that includes plots (702, 704, 708, 806, 810). Plots (702, 704, 708) are the same as those shown and described in FIG. 16. As described above with reference to FIG. 16, plots (706, 710) are shown with tissue not compressing. Conversely, FIG. 17 shows plots (806, 810) experiencing tissue compression. Plot (806) corresponds to the compression of biasing feature (206, 334) in a loaded state. Plot (810) refers to the distance between jaws (182, 184) or the distance between jaws (328, 330) in the loaded state. Similarly, plot (810) may be determined by subtracting plot (806) corresponding to the sensed compression of biasing feature (206, 334) from plot (702) corresponding to the sensed position of clamping drive train (204, 342). As a result, obtaining sensor information for plot (702) and plot (806) allows for estimation of plot (810).

FIG. 18 shows an exemplary graph (900) that includes plots (902, 904, 906). Similar to plot (702), plot (902) corresponds to the position of handle assembly (220, 402), which may be determined by clamp closure sensors (218) sensing clamping drive train (204, 342) over time. Plot (904) corresponds to the compression of biasing feature (206, 334) over time. Plot (906) refers to the distance between jaws (182, 184) or the distance between jaws (328, 330) over time. Once triggers (126, 316) are fully actuated at point (908), the distance remains constant. FIG. 18 shows three phases that include a pre-energy application phase (910), an energy application phase (912), and a post-energy application phase (912). As tissue becomes denatured during energy application phase (912), the compression of biasing feature (206, 334) decreases as the tissue provides reduces resistance. As a result, plot (906) decreases.

FIG. 19 shows an exemplary graph (1000) that includes plots (1002, 1004, 1006). Similar to plot (902), plot (1002) corresponds to the position of handle assembly (220, 402), which may be determined by clamp closure sensors (218) sensing clamping drive train (204, 342) over time. Once triggers (126, 316) are fully actuated at point (1008), the distance remains constant. Plot (1004) corresponds to the compression of biasing feature (206, 334) over time. Plot (1006) refers to the distance between jaws (182, 184) or the distance between jaws (328, 330) over time. Unlike plot (906), plot (1006) shows tissue slipping out along plot segment (1010) from between jaw (182, 184, 328, 330) as jaws (182, 184, 328, 330) are overstuffed. It may be beneficial to alert the user (e.g., using display (226)) that tissue is slipping out from between jaws (182, 184, 328, 330).

### E. Exemplary Method

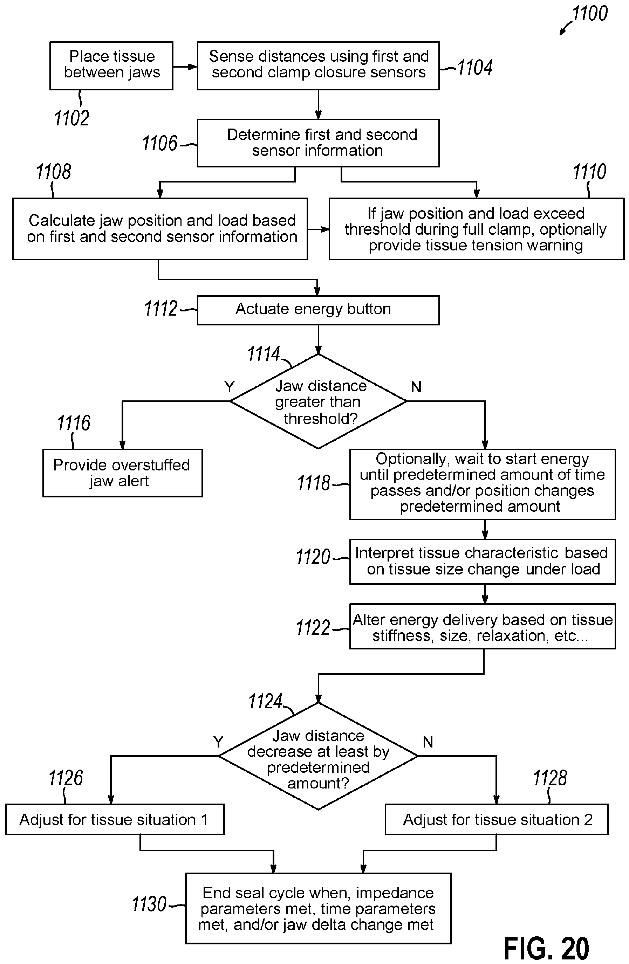

A method (1100) of operating electrosurgical instruments (200, 300, 400) is described below with reference to FIG. 20. At step (1102), tissue (T) may be placed between jaws (182, 184, 328, 330) of electrosurgical instruments (200, 300, 400) when jaws (182, 184) are in an open configuration. At step (1104), the movement of clamp drive train (204, 342) is sensed using clamp closure sensors (218, 454) and the compression of biasing feature (206, 334) is sensed using clamp closure sensors (224, 454). This sensing may be continuously performed until the energy application is completed. In some versions, at step (1104), jaw closure trigger (316, 416) is actuated to a fully closed configuration (e.g., where switch (332) is completed/closed). This actuation of trigger (126, 316, 416) biases biasing feature (206, 334) to clamp tissue (T) between jaws (182, 184) toward the closed configuration. The act of biasing may include compressing compression spring (208) or wave spring assembly (336) to clamp tissue (T) between jaws (182, 184, 328, 330) in the closed configuration.

At step (1106), this movement of clamping drive train (204, 342) and biasing of compression spring (208) or wave spring assembly (336) may be tracked using one or more clamp closure sensors (218, 224, 346, 452, 454). Sensors (218, 224, 346, 452, 454) may produce first and second sensor information. The distance (d1, d2, d3) between clamp closure sensor (218) and sensor receiver mechanism (216) provides information as to the translation of clamping drive train (204, 342). The distance (D1, D2, D3) between clamp closure sensor (224, 346, 454) and sensor receiver mechanism (225, 344, 444) provides information as to the compression of compression spring (208) or wave spring assembly (336). As shown in FIGS. 7A-7C regarding compression spring (208) or FIGS. 11A-11C regarding wave spring assembly (336), the distance compression spring (208) or wave spring assembly (336) compresses corresponds to the magnitude that distance (D1, D2, D3) increases as at least a portion of clamping drive train (204, 342) (shown schematically) is forced proximally. As shown, distance (D3) may be smaller than distance (D2) as energy is distances (D1, D2, D3) may be communicated to control unit (202, 324, 424). Similarly, as shown in FIG. 13, clamp closure sensor (452) measures distance (D4) using sensor receiver mechanism (444), and clamp closure sensor (454) measures distance (D5) using sensor receiver mechanism (444).

At step (1108), method (1100) includes estimating the tissue load exerted on jaws (182, 184, 328, 330) and a jaw position (jaw distance (X) and/or jaw angle(a)) may be based on the act of tracking. For example, control unit (202, 324, 424) may calculate jaw position and load based on the movement of biasing feature (206, 334) and the movement of sensor receiver mechanism (216, 344, 444). Control unit (202, 324, 424) may utilize a look up table or calculation to interoperate jaw load and jaw position (jaw distance (X) and/or jaw angle(a)) due to compression of biasing feature (206, 334, 434).

At step (1110), if jaw load and/or jaw position (jaw distance (X) and/or jaw angle(a)) exceed a predetermined threshold during full clamping, control unit (202, 324, 424) may optionally provide tissue tension warning. Clamp closure sensor (224, 346, 452, 454) reads movement of clamping drive chain (204, 342) using sensor receiver mechanism (216, 344, 444).

At step (1112), button (130, 312, 412) may be actuated. At step (1114), control unit (202, 324, 424) determines whether distance (X) between jaws (182, 184, 328, 330) is greater than a predetermined threshold. At step (1116), if distance (X) between jaws (182, 184, 328, 330) is greater than the predetermined threshold, control unit (202, 324, 424) may provide an overstuffed jaw alert. The overstuffed jaw alert may include one or more of an audible alert, a visual alert, or a tactile alert (e.g., vibration of body (122, 308, 408)).

If distance (X) between jaws (182, 184, 328, 330) is not greater than the predetermined threshold, control unit (202, 324, 424) proceeds with sealing. Optionally, at step (1118), control unit (202, 324, 424) may delay the application of energy until a predetermined duration of time elapses and/or the position of jaws (184, 330) changes a predetermined amount. At step (1120), control unit (202, 324, 424) interprets one or more tissue characteristics based on tissue size change under load. At step (1122), at least one sealing parameter may be altered in response to the act of estimating. Control unit (202, 324, 424) alters sealing parameters as needed before and during sealing as a response to the clamp closure sensor (224) and spring load. For example, control unit (202, 324, 424) alters energy delivery based on tissue stiffness, size, relaxation, etc.

At step (1124), control unit (202, 324, 424) determines whether distance (X) between jaws (182, 184, 328, 330) decreases at least by a predetermined amount. At step (1126), if distance (X) decreases at least by the predetermined amount, control unit (202, 324, 424) adjusts for the new tissue situation (e.g., tissue situation 1). At step (1128), if distance (X) between jaws (182, 184, 328, 330) does not decrease at least by the predetermined amount, then control unit (202, 324, 424) adjusts for the new tissue situation (e.g., tissue situation 2).

At step (1130), the sealing cycle may be completed when predetermined impedance parameters are satisfied, predetermined time parameter are satisfied, and/or predetermined jaw delta change are satisfied. Steps (1124, 1126, 1128, 1130) may be repeated one or more times.

### III. Exemplary Combinations

The following examples relate to various non-exhaustive ways in which the teachings herein may be combined or applied. It should be understood that the following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1

An electrosurgical instrument, comprising: (a) a shaft assembly; (b) an end effector that extends distally from the shaft assembly, the end effector comprising: (i) an energized feature configured to apply energy to a tissue, (ii) a first jaw, and (iii) a second jaw configured to selectively move relative to the first jaw from an open configuration configured to receive the tissue to a closed configuration configured to clamp the tissue; (c) an input actuator operatively connected to the end effector and configured to selectively move from an unactuated position to an actuated position to thereby move the first and second jaws from the open configuration toward the closed configuration; (d) a biasing feature operatively connected between the input actuator and the end effector and configured to resiliently deflect for limiting force transmitted therethrough;(e) a first clamp closure sensor configured to produce first sensor information relating to movement of the input actuator in response to the input actuator being moved from the unactuated position to the actuated position; (f) a second clamp closure sensor configured to produce second sensor information relating to deflection of the biasing feature in response to a tissue load force between the first and second jaws; and (g) a control unit operatively connected with the first and second clamp closure sensors, wherein, based on each of the first and second sensor information produced by the first and second clamp closure sensors, the control unit is configured to determine: (i) the tissue load force exerted on the end effector, and (ii) a position of one of the first and second jaws relative to the other of the first and second jaws.

### Example 2

The electrosurgical instrument of Example 1, wherein the first clamp closure sensor is disposed proximal to the biasing feature.

### Example 3

The electrosurgical instrument of Example 1, wherein the first clamp closure sensor is disposed distal to the biasing feature.

### Example 4

The electrosurgical instrument of any of Examples 1 through 3, wherein the first clamp closure sensor includes at least one of an optical reflective sensor, an optical linear encoder, a magnetic sensor, or a capacitive sensor.

### Example 5

The electrosurgical instrument of any of Examples 1 through 4, further comprising a clamping drive train connected between the input actuator and the end effector and configured to operatively move the first and second jaws from the open configuration toward the closed configuration, wherein the first clamp closure sensor is configured to sense movement of the clamping drive train.

### Example 6

The electrosurgical instrument of Example 5, further comprising a first sensor receiver mechanism configured to be sensed by the first clamp closure sensor, wherein the clamping drive train includes an actuation rod configured to translate within the shaft assembly to move the first and second jaws from the open configuration toward the closed configuration, wherein either the first clamp closure sensor or a first sensor receiver mechanism is operatively coupled with the actuation rod.

### Example 7

The electrosurgical instrument of Example 6, wherein the first clamp closure sensor or the first sensor receiver mechanism is coupled with a proximal end of the actuation rod.

### Example 8

The electrosurgical instrument of Example 6, wherein the first sensor receiver mechanism is coupled with a proximal end of the actuation rod.

### Example 9

The electrosurgical instrument of any of Examples 6 through 8, further comprising a slip ring in electrical communication with the first clamp closure sensor, wherein the slip ring is configured to allow for rotation of the first clamp closure sensor or the first sensor receiver mechanism relative to the other of the first clamp closure sensor or the first sensor receiver mechanism.

### Example 10

The electrosurgical instrument of any of Examples 6 through 9, wherein the first sensor receiver mechanism comprises at least one of a magnet or a reflector.

### Example 11

The electrosurgical instrument of any of Examples 6 through 10, wherein the first sensor receiver mechanism comprises a magnet, wherein the first clamp closure sensor comprises a Hall Effect sensor configured to sense the magnet.

### Example 12

The electrosurgical instrument of any of Examples 1 through 11, further comprising a handle assembly extending proximally from the shaft assembly, wherein the handle assembly includes a display configured to indicate to a user whether the tissue load force exceeds a predetermined value corresponding to a partial sealing using the first and second jaws of the electrosurgical instrument.

### Example 13

The electrosurgical instrument of any of Examples 1 through 12, wherein the first clamp closure sensor includes a first reflective optocoupler, wherein the second clamp closure sensor includes a second reflective optocoupler.

### Example 14

The electrosurgical instrument of any of Examples 1 through 13, wherein the energized feature includes at least one of an ultrasonic blade or at least one electrode.

### Example 15

The electrosurgical instrument of any of Examples 1 through 14, wherein the position of the first jaw relative to the second jaw includes a jaw angle defined between the first and second jaws.

### Example 16

An electrosurgical instrument, comprising: (a) a shaft assembly; (b) an end effector that extends distally from the shaft assembly, the end effector comprising: (i) a first jaw that includes an energized feature configured to apply energy to a tissue, and (ii) a second jaw, wherein at least one of the first and second jaws is configured to pivot relative to the other of the first and second jaws between an open configuration configured to receive the tissue and a closed configuration configured to clamp the tissue; (c) an input actuator configured to selectively move from an unactuated position to an actuated position; (d) a clamping drive train connected between the input actuator and the end effector, wherein the clamping drive train includes an actuation rod configured to translate within the shaft assembly to move the first and second jaws from the open configuration toward the closed configuration, wherein the actuation rod includes a proximal end; (e) a first clamp closure sensor configured to sense movement of the proximal end of the actuation rod and produce first sensor information as the clamping drive train moves the first and second jaws from the open configuration toward the closed configuration in response to the input actuator being moved from the unactuated position to the actuated position; (f) a biasing feature operatively connected between the input actuator and the end effector and configured to resiliently deflect for limiting force transmitted therethrough; (g) a second clamp closure sensor configured to generate second sensor information relating to movement of the biasing feature in response to a tissue load force; and (h) a control unit configured to use the first and second sensor information to determine at least one of: (i) the tissue load force exerted on the end effector, or (ii) a position of one of the first and second jaws relative to the other of the first and second jaws.

### Example 17

The electrosurgical instrument of Example 16, further comprising a first sensor receiver mechanism configured to be sensed by the first clamp closure sensor, wherein the first clamp closure sensor or the first sensor receiver mechanism is coupled with a proximal end of the actuation rod.

### Example 18

An electrosurgical instrument, comprising: (a) a shaft assembly; (b) an end effector that extends distally from the shaft assembly, the end effector comprising: (i) an energized feature configured to apply energy to a tissue, (ii) a first jaw, and (iii) a second jaw configured to selectively move relative to the first jaw from an open configuration configured to receive the tissue to a closed configuration configured to clamp the tissue; (c) an input actuator operatively connected to the end effector and configured to selectively move from an unactuated position to an actuated position to thereby move the first and second jaws from the open configuration toward the closed configuration; (d) a biasing feature operatively connected between the input actuator and the end effector and configured to resiliently deflect for limiting force transmitted therethrough;(e) a first clamp closure sensor configured to produce first sensor information relating to movement of the input actuator in response to the input actuator being moved from the unactuated position to the actuated position; (f) a second clamp closure sensor configured to produce second sensor information relating to deflection of the biasing feature in response to a tissue load force between the first and second jaws; and (g) a control unit operatively connected with the first and second clamp closure sensors, wherein, based on each of the first and second sensor information produced by the first and second clamp closure sensors, the control unit is configured to determine: (i) the tissue load force exerted on the end effector, and (ii) a position of one of the first and second jaws relative to the other of the first and second jaws.

### Example 19

The electrosurgical instrument of any of Examples 1 through 18, wherein the first clamp closure sensor includes at least one of a Hall Effect sensor, a light sensor, a resistive sensor, a capacitive sensor, or a linear variable differential transformer.

### Example 20

The electrosurgical instrument of any one or more of Examples 1 through 11 and Examples 13 through 19, further comprising a handle assembly, wherein the first clamp closure sensor is translatably coupled with the handle assembly.

### Example 21

The electrosurgical instrument of any one or more of Examples 18 through 20, further comprising a clamping drive train connected between the input actuator and the end effector and configured to operatively move the first and second jaws from the open configuration toward the closed configuration, wherein the first clamp closure sensor is configured to sense movement of the clamping drive train.

### Example 22

The electrosurgical instrument of Example 21, further comprising a sensor receiver mechanism translatably coupled with the clamping drive train, wherein the first clamp closure sensor is configured to emit a signal that is influenced by the sensor receiver mechanism to generate the clamp closure sensor information.

### Example 23

The electrosurgical instrument of Example 22, wherein the sensor receiver mechanism comprises at least one of a magnet or a reflector.

### Example 24

The electrosurgical instrument of any one or more of Examples 1 through 6, further comprising a second clamp closure sensor, wherein the first clamp closure sensor includes a first reflective optocoupler, wherein the second clamp closure sensor includes a second reflective optocoupler.

### Example 25

The electrosurgical instrument of any one or more of Examples 18 through 24, wherein the energized feature includes at least one of an ultrasonic blade or at least one electrode.

### Example 26

The electrosurgical instrument of Example 25, further comprising an ultrasonic transducer configured to provide ultrasonic energy to the ultrasonic blade.

### Example 27

The electrosurgical instrument of Example 26, the at least one electrode further comprising: (i) a first electrode surface secured relative to the first jaw, (ii) a second electrode surface positioned to face the first electrode surface when the first and second jaws are in the closed configuration, wherein the first and second electrode surfaces are operable to apply RF energy to the tissue.

### Example 28

The electrosurgical instrument of any one or more of Examples 18 through 27, wherein the biasing feature includes a spring, wherein the control unit is configured to use the clamp closure sensor information to measure compression of the spring to determine at least one of the tissue load force or the position of the first jaw relative to the second jaw based on the clamp closure sensor information with the input actuator in the actuated position.

### Example 29

The electrosurgical instrument of any one or more of Examples 18 through 28, wherein the end of stroke feature includes an end of stroke switch configured to complete a circuit in the closed configuration.

### Example 30

The electrosurgical instrument of Example 29, further comprising a handle assembly that includes a pistol grip, wherein the end of stroke switch is positioned within the pistol grip.

### Example 31

The electrosurgical instrument of any one or more of Examples 18 through 30, wherein the control unit is configured to determine the tissue load force and the position of the first jaw relative to the second jaw based on the clamp closure sensor information with the input actuator in the actuated position.

### Example 32

The electrosurgical instrument of Example 31, wherein the position of the first jaw relative to the second jaw includes a jaw angle defined between the first and second jaws.

### Example 33

An electrosurgical instrument, comprising: (a) a shaft assembly; (b) an end effector that extends distally from the shaft assembly, the end effector comprising: (i) a first jaw that includes an energized feature configured to apply energy to a tissue, and (ii) a second jaw, wherein at least one of the first and second jaws is configured to pivot relative to the other of the first and second jaws between an open configuration configured to receive the tissue and a closed configuration configured to clamp the tissue; (c) an input actuator operatively connected to the end effector and configured to selectively move from an unactuated position to an actuated position to thereby move the first and second jaws from the open configuration toward the closed configuration; (d) an end of stroke feature configured to detect that the input actuator is in the actuated position; (e) a biasing feature operatively connected between the input actuator and the end effector and configured to resiliently deflect for limiting force transmitted therethrough (f) a sensor receiver mechanism operatively coupled with the biasing feature; (g) a clamp closure sensor configured to emit a signal that is influenced by the sensor receiver mechanism to generate clamp closure sensor information relating to movement of the biasing feature in response to the tissue load force; and (h) a control unit configured to use the clamp closure sensor information to determine: (i) the tissue load force exerted on the end effector, and (ii) a position of at least one of the first and second jaws.

### Example 34

A method of using an electrosurgical instrument, comprising: (a) receiving a tissue between first and second jaws of an end effector of the electrosurgical instrument; (b) moving an actuator from a non-actuated position to an actuated position to bias a biasing feature while clamping the tissue between the first and second jaws toward a closed configuration; (c) tracking a position of a clamping drive train connected between the actuator and the end effector using a first clamp closure sensor; (d) tracking a position of at least a portion of the biasing feature using a second clamp closure sensor; and (e) determining a tissue load force exerted on the first and second jaws and a position of at least one of the first and second jaws based on the tracking of the position of the first and second clamp closure sensors.

### Example 35

The method of Example 34, further comprising providing an alert to the user to readjust the position of the first and second jaws relative to the tissue in response to determining the tissue load force exceeds a predetermined value corresponding to a partial sealing using the first and second jaws of the electrosurgical instrument.

### Example 36

The method of any of Examples 34 through 35, wherein tracking the position of the actuator further comprises tracking the position a proximal end of an actuation rod of the clamping drive train using the first clamp closure sensor.

### Example 37

A method of using an electrosurgical instrument, comprising: (a) receiving a tissue between first and second jaws of the electrosurgical instrument; (b) moving an actuator from a non-actuated position to an actuated position to bias a biasing feature while clamping the tissue between the first and second jaws toward a closed configuration; (c) determining the actuator is in the actuated position using an end of stroke feature; (d) tracking a position of at least a portion of the biasing feature using a clamp closure sensor with the actuator is in the actuated position; and (e) determining a tissue load force exerted on the first and second jaws or a position of at least one of the first and second jaws based on the tracking of the position of the at least the portion of the biasing feature with the actuator is in the actuated position.

### Example 38

The method of any of Examples 34 through 37, wherein the act of determining further comprises determining the tissue load force exerted on the first and second jaws and a position of at least one of the first and second jaws based on the tracking of the position of the at least the portion of the biasing feature.

### Example 39

The method of any one or more of Examples 38 through 38, wherein the biasing feature includes a spring, wherein the act of biasing further comprises compressing the spring to clamp the tissue between the first and second jaws toward the closed configuration, wherein the act of biasing further comprises tracking the compression of the spring using the clamp closure sensor.

### Example 40

The method of any one or more of Examples 34 through 39, further comprising altering at least one sealing parameter in response to the act of determining the tissue load force.

### IV. Miscellaneous

It should be understood that any of the versions of the instruments described herein may include various other features in addition to or in lieu of those described above. By way of example only, any of the devices herein may also include one or more of the various features disclosed in any of the various references that are incorporated by reference herein. Various suitable ways in which such teachings may be combined will be apparent to those of ordinary skill in the art.

While the examples herein are described mainly in the context of electrosurgical instruments, it should be understood that various teachings herein may be readily applied to a variety of other types of devices. By way of example only, the various teachings herein may be readily applied to other types of electrosurgical instruments, tissue graspers, tissue retrieval pouch deploying instruments, surgical staplers, surgical clip appliers, ultrasonic surgical instruments, etc. It should also be understood that the teachings herein may be readily applied to any of the instruments described in any of the references cited herein, such that the teachings herein may be readily combined with the teachings of any of the references cited herein in numerous ways. Other types of instruments into which the teachings herein may be incorporated will be apparent to those of ordinary skill in the art.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions or other disclosure material set forth herein will only be incorporated to the extent that no conflict arises between that incorporated material and the existing disclosure material.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of U.S. Pat. No. 6,783,524, entitled "Robotic Surgical Tool with Ultrasound Cauterizing and Cutting Instrument," published August 31, 2004, the disclosure of which is incorporated by reference herein, in its entirety.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometries, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. An electrosurgical instrument, comprising:
(a) a shaft assembly;
(b) an end effector that extends distally from the shaft assembly, the end effector comprising:
(i) an energized feature configured to apply energy to a tissue,
(ii) a first jaw, and
(iii) a second jaw configured to selectively move relative to the first jaw from an open configuration configured to receive the tissue to a closed configuration configured to clamp the tissue;
(c) an input actuator operatively connected to the end effector and configured to selectively move from an unactuated position to an actuated position to thereby move the first and second jaws from the open configuration toward the closed configuration;
(d) a biasing feature operatively connected between the input actuator and the end effector and configured to resiliently deflect for limiting force transmitted therethrough;
(e) a first clamp closure sensor configured to produce first sensor information relating to movement of the input actuator in response to the input actuator being moved from the unactuated position to the actuated position;
(f) a second clamp closure sensor configured to produce second sensor information relating to deflection of the biasing feature in response to a tissue load force between the first and second jaws; and
(g) a control unit operatively connected with the first and second clamp closure sensors, wherein, based on each of the first and second sensor information produced by the first and second clamp closure sensors, the control unit is configured to determine:
(i) the tissue load force exerted on the end effector, and
(ii) a position of one of the first and second jaws relative to the other of the first and second jaws.

2. The electrosurgical instrument of claim 1, wherein the first clamp closure sensor is disposed proximal to or distal to the biasing feature.

3. The electrosurgical instrument of claim 1 or claim 2, wherein the first clamp closure sensor includes at least one of an optical reflective sensor, an optical linear encoder, a magnetic sensor, or a capacitive sensor.

4. The electrosurgical instrument of any preceding claim, further comprising a clamping drive train connected between the input actuator and the end effector and configured to operatively move the first and second jaws from the open configuration toward the closed configuration, wherein the first clamp closure sensor is configured to sense movement of the clamping drive train.

5. The electrosurgical instrument of claim 4, further comprising a first sensor receiver mechanism configured to be sensed by the first clamp closure sensor, wherein the clamping drive train includes an actuation rod configured to translate within the shaft assembly to move the first and second jaws from the open configuration toward the closed configuration, wherein either the first clamp closure sensor or a first sensor receiver mechanism is operatively coupled with the actuation rod.

6. The electrosurgical instrument of claim 5, wherein the first clamp closure sensor or the first sensor receiver mechanism is coupled with a proximal end of the actuation rod.

7. The electrosurgical instrument of claim 5, further comprising a slip ring in electrical communication with the first clamp closure sensor, wherein the slip ring is configured to allow for rotation of the first clamp closure sensor or the first sensor receiver mechanism relative to the other of the first clamp closure sensor or the first sensor receiver mechanism.

8. The electrosurgical instrument of claim 5, wherein the first sensor receiver mechanism comprises at least one of a magnet or a reflector.

9. The electrosurgical instrument of claim 5, wherein the first sensor receiver mechanism comprises a magnet, wherein the first clamp closure sensor comprises a Hall Effect sensor configured to sense the magnet.

10. The electrosurgical instrument of any preceding claim, further comprising a handle assembly extending proximally from the shaft assembly, wherein the handle assembly includes a display configured to indicate to a user whether the tissue load force exceeds a predetermined value corresponding to a partial sealing using the first and second jaws of the electrosurgical instrument.

11. The electrosurgical instrument of any preceding claim, wherein the first clamp closure sensor includes a first reflective optocoupler, wherein the second clamp closure sensor includes a second reflective optocoupler.

12. The electrosurgical instrument of any preceding claim, wherein the energized feature includes at least one of an ultrasonic blade or at least one electrode.

13. The electrosurgical instrument of any preceding claim, wherein the position of the first jaw relative to the second jaw includes a jaw angle defined between the first and second jaws.

14. An electrosurgical instrument, comprising:
(a) a shaft assembly;
(b) an end effector that extends distally from the shaft assembly, the end effector comprising:
(i) a first jaw that includes an energized feature configured to apply energy to a tissue, and
(ii) a second jaw, wherein at least one of the first and second jaws is configured to pivot relative to the other of the first and second jaws between an open configuration configured to receive the tissue and a closed configuration configured to clamp the tissue;
(c) an input actuator configured to selectively move from an unactuated position to an actuated position;
(d) a clamping drive train connected between the input actuator and the end effector, wherein the clamping drive train includes an actuation rod configured to translate within the shaft assembly to move the first and second jaws from the open configuration toward the closed configuration, wherein the actuation rod includes a proximal end;
(e) a first clamp closure sensor configured to sense movement of the proximal end of the actuation rod and produce first sensor information as the clamping drive train moves the first and second jaws from the open configuration toward the closed configuration in response to the input actuator being moved from the unactuated position to the actuated position;
(f) a biasing feature operatively connected between the input actuator and the end effector and configured to resiliently deflect for limiting force transmitted therethrough;
(g) a second clamp closure sensor configured to generate second sensor information relating to movement of the biasing feature in response to a tissue load force; and
(h) a control unit configured to use the first and second sensor information to determine at least one of:
(i) the tissue load force exerted on the end effector, or
(ii) a position of one of the first and second jaws relative to the other of the first and second jaws.

15. The electrosurgical instrument of claim 14, further comprising a first sensor receiver mechanism configured to be sensed by the first clamp closure sensor, wherein the first clamp closure sensor or the first sensor receiver mechanism is coupled with a proximal end of the actuation rod.
